(19) Europäisches Patentamt
European Patent Office
Office européen des brevets

(11) Numéro de publication: **0 360 626**
**A1**

(12) **DEMANDE DE BREVET EUROPEEN**

(21) Numéro de dépôt: **89401681.5**

(22) Date de dépôt: **15.06.89**

(51) Int. Cl.[5]: **C 07 H 19/067**
C 07 H 19/167, C 07 H 21/02

(30) Priorité: **20.09.88 FR 8812264**

(43) Date de publication de la demande:
**28.03.90 Bulletin 90/13**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75007 Paris (FR)**

(72) Inventeur: **Rayner, Bernard Chênes Colombière, G 68**
**180 avenue d'Occitanie**
**F-34100 Montpellier (FR)**

**Imbach, Jean-Louis Chemin Clos des Oliviers**
**1108 Rue de Las Sorbes**
**F-34000 Montpellier (FR)**

(74) Mandataire: **Warcoin, Jacques et al**
**Cabinet Régimbeau 26, avenue Kléber**
**F-75116 Paris (FR)**

(54) **Procédé de synthèse d'oligoribonucléotides alpha et composés utiles dans le procédé.**

(57) La présente invention a pour objet un procédé de synthèse de composés oligoribonucléotides alpha caractérisé en ce qu'on effectue une synthèse du type au phosphoramidite sur support solide, ainsi que des composés utiles dans ledit procédé, et les composés obtenus.

Selon l'invention, on assemble des monomères ribonucléosides alpha protégés, substitués par des groupes phosphoramidite en 3′, la fonction hydroxyle en 2′ du ribose dudit monomère étant protégée par le groupe Ctmp ou TBDMS.

EP 0 360 626 A1

## Description

### PROCEDE DE SYNTHESE D'OLIGORIBONUCLEOTIDES ALPHA ET COMPOSES UTILES DANS LE PROCEDE

La présente invention concerne un nouveau procédé de synthèse de composés oligoribonucléotides alpha et les composés obtenus par le procédé. De tels composés ont été décrits dans l'état de la technique, dans la demande de brevet FR 87 04339 et la publication internationale N° 88/04301 au nom de la présente demanderesse, mais leur procédé de préparation par une méthode au phosphotriester en solution n'était connu que pour de composés comportant uniquement les bases U et C.

Ces composés oligoribonucléotides alpha consistent de des enchainements de nucléotides d'acides ribonucléiques possédant la configuration anomérique alpha non naturelle, par opposition à la configuration naturelle bêta.

Ces composés peuvent être facultativement liés à un agent effecteur, notamment par une liaison covalente. Ces agents effecteurs sont maintenant bien connus dans la technique touchant aux acides nucléiques. Il s'agit principalement de radicaux intercalants, de radicaux chimiques porteurs d'une fonction réagissant directement ou indirectement avec les chaines de nucléotides ou de marqueurs dont la présence permet une détection facile, les radicaux chimiques pouvant également constituer des agents intercalants.

Plus précisément, la présente invention a pour objet un procédé de préparation par synthèse sur support solide par la méthode au phosphoramidite, de composés oligoribonucléotides alpha.

Des synthèses par la méthode au phosphoramidite sur support solide ont déjà été proposées pour préparer des composés oligodéoxyribonucléotides alpha, notamment dans la demande de brevet FR 87 04339. En revanche, les composés oligoribonucléotides alpha n'ont jamais été préparés par synthèse sur support solide dans l'état de la technique, notamment à l'aide de synthétiseurs automatisés.

La préparation des composés oliribonucléotides alpha a été proposée dans l'état de la technique notamment dans la demande de brevet FR 87 04339, selon une technique en solution, par le procédé dit au phosphotriester en solution.

La présente invention apporte désormais une solution au problème clé de la protection des groupes hydroxyle en position 2′ des riboses dans le cadre d'une synthèse sur support solide, en proposant les deux groupes TBDMS (Terbutyldiméthylsilyl) et Ctmp : (chloro-2 méthyl-4 phényl)-1 méthoxy-4 pipéridinyl-4.

Le groupement Ctmp présente l'intérêt d'être compatible avec l'utilisation du groupement Dmtr (dimethoxytrityl) utilisé pour la protection des groupements hydroxyle en 5′ du ribose, car il reste intact dans les conditions de déprotection de celui-ci dans un procédé de préparation sur support solide contrairement par exemple au groupement méthoxy-4 tétrahydropyrannyl-4 (Mthp).

L'autre groupement protecteur proposé selon la présente invention, à savoir le TBDMS, offre l'avantage d'une synthèse du monomère réactif plus facile et d'une élimination très aisée par un traitement aux ions fluorures, notamment par le fluorure de tétrabutylammonium dans le THF anhydre.

Le procédé en phase solide selon la méthode au phosphoramidite, objet de la présente invention, comporte les étapes essentielles suivantes :

- On immobilise un dérivé ribonucléoside alpha protégé, par l'intermédiaire d'une de ses fonctions hydroxyle en 2′ ou 3′ sur un support solide.
- On assemble sur ce support dans un réacteur synthétiseur manuel ou automatique la chaîne d'oligoribonucléotide alpha protégée par condensation de monomères constitués de ribonucléosides alpha protégés substitués par des groupes phosphoramidites en 3′, la fonction hydroxyle en 2′ des riboses étant protégées par les groupes Ctmp ou TBDMS.
- Les oligoribonucléotides alpha sont obtenus après décrochage de l'oligomère obtenu du support et élimination des groupes protecteurs.

De façon appropriée, l'assemblage de l'oligoribonucléotide alpha se fait par condensation, en présence d'un agent activateur, desdits monomères entre leur fonction en 3′ et la fonction hydroxyle-5′ du composé ribonucléoside alpha immobilisé pour le premier monomère ou d'un composé intermédiaire polyribonucléotide alpha protégé fixé sur ledit composé ribonucléoside alpha immobilisé pour les monomères suivants.

Notamment, l'agent activateur pour la condensation desdits monomeres peut être choisi parmi le tétrazole et ses dérivés, tels que le paranitro-phényl tétrazole.

Avantageusement, le groupe phosphoramidite en 3′ desdits monomères est un groupe N,N dialkylamino-phosphoramidite de formule

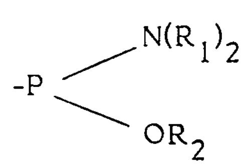

(I)

avec $R_1$ qui représente un alkyle en $C_1$ à $C_7$ éventuellement substitué identique ou différent ,tel que $CH_3$, $(CH_3)_2CH$

$R_2$ qui représente un alkyle en $C_1$ à $C_7$ éventuellement substitué, tel que $CH_3$, $CH_2CH_2CN$.

En particulier, le groupe phosphoramidite en 3′ desdits monomères est le diisopropylaminophosphoramidite de méthyle ($R_1$ = $(CH_3)_2CH$ et $R_2$ = $CH_3$) ou le diisopropylaminophosphoramidite de cyano-2 éthyle ($R_1$ = $(CH_3)_2CH$ et $R_2$ = $CH_2CH_2CN$).

De façon appropriée, les groupes hydroxyles desdits monomères et dudit composé ribonucléoside alpha immobilisé peuvent être protégés en 5′ par le groupe Dmtr.

Le composé ribonucléoside alpha immobilisé peut être fixé sur le support solide via un groupe divalent succinyle entre un de ses groupements OH en 2′ ou 3′ qui se trouve ainsi estérifié, et un groupement amino du support.

Le support solide peut être constitué par une longue chaîne alkylamine fixée sur un polymère, un gel de silice ou des billes de verre poreuses.

Lorsque l'on veut préparer un oligoribonucléotide alpha lié à un radical effecteur, on peut coupler la fonction hydroxylée 5′ terminal de l'oligoribonucléotide alpha protégé lié au support solide, avec un monomère substitué par un radical effecteur tel qu'un groupe intercalant, un groupe chimiquement activable ou un groupe photoactivable. L'oligoribonucléotide alpha protégé lié au radical effecteur est ensuite décroché du support solide puis des groupes protecteurs sont éliminés.

Selon l'invention, il est également possible de coupler la fonction hydroxyle 3′ ou 5′ terminal de l'oligoribonucléotide alpha préparé après décrochage et élimination des groupes protecteurs avec un radical effecteur selon les techniques connues de l'homme de l'art en particulier via une chaîne alcoylène.

Ainsi, il est possible selon le procédé objet de la présente invention de préparer des composés de formule générale

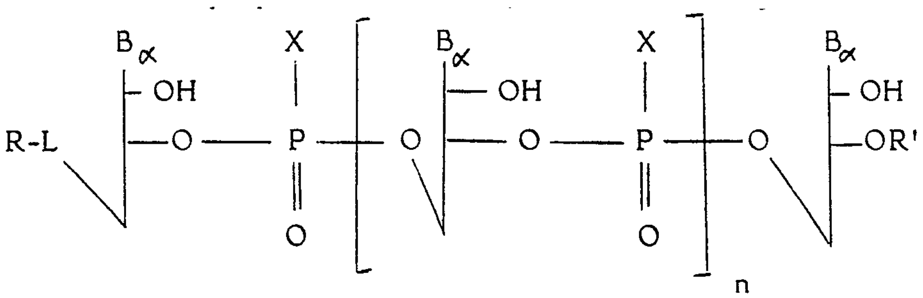

dans laquelle :

Les radicaux B peuvent être identiques ou différents et représentent chacun une base d'un acide ribonucléique éventuellement modifiée et rattachée au cycle glycosidique selon une configuration anomérique alpha non naturelle ;

Les radicaux X peuvent être identiques ou différents et représentent chacun un oxoanion $O^-$, un thioanion $S^-$, un groupe alkyle, un groupe alcoxy, aryloxy, un groupe aminoalkyle, un groupe aminoalcoxy, un groupe thioalkyle, Y-Z.

R et R′, qui peuvent être identiques ou différents, représentent chacun un atome d'hydrogène ou un groupement -Y-Z.

Y représente un radical alcoylène droit ou ramifié -alk- ou un radical choisi parmi 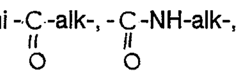

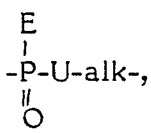 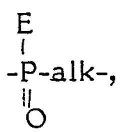

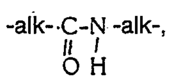

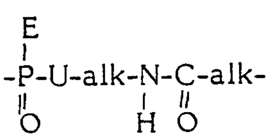 et 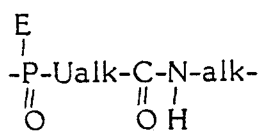

avec U = O, N ou S.

ou bien un radical -Y″-O-Y′ où Y″ ou Y′ peuvent avoir les significations données pour Y ;

E peut avoir les mêmes significations que X, excepté Y-Z.

Z est un radical correspondant à un agent effecteur.

n est un nombre entier y compris O.

L représente un atome d'oxygène, un atome de soufre ou un groupe -NH-.

Dans cette formule générale, ou utilise la représentation condensée des nucléosides suivante :

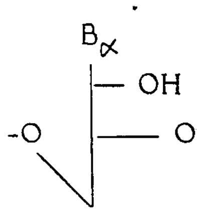

qui correspond à la formule développée :

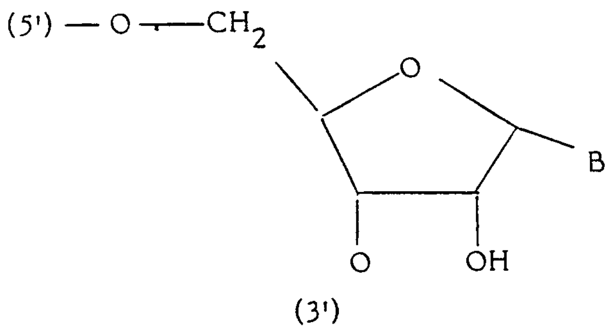

sur laquelle ont été mentionnées les extrémités (3′) et (5′).

Il convient de remarquer que la formule générale représente un enchaînement de nucléotides qui peuvent être identiques ou différents, n indiquant simplement le nombre de nucléotides compris dans la molécule ; n est de préférence un nombre compris entre 1 et 100, et de préférence encore entre 1 et 50.

Les radicaux effecteurs correspondent à des agents effecteurs qui sont des composés connus dans les techniques touchant aux acides nucléiques. Il s'agit par exemple des composés capables de "s'intercaler" dans la structure des ADN ou des ARN.

Ces agents d'intercalation sont, engénéral, constitués par des composés polycycliques ayant une configuration plane tels que l'acridine, la furocoumarine, la daunomycine, la 1,10-phénanthroline, la phénanthridinium, les prophyrines, les dérivés de la dipyrido (1,2-a : 3′, 2′-d) imidazole, l'ellipticine ou l'ellipticinium et leurs dérivés.

Ces agents effecteurs peuvent aussi être des radicaux chimiques réactifs tels que des radicaux chimiques de scission, c'est-à-dire que ces radicaux peuvent directement ou indirectement réagir pour scinder une chaîne de nucléotides. De préférence, ces radicaux chimiques réactifs seront activables, par exemple par voie chimique ou photochimique. Les groupes réactifs de scission activables sont, par exemple, des dérivés de composés tels que :
l'acide éthylène-diamine-tétracétique,
l'acide diéthylène-triamine-pentaacétique,
les porphyrines,
la 1,10-phénanthroline, le psoralène et autres groupes aromatiques absorbant les radiations du proche U.V. et du visible.

Ces groupements chimiquement activables en présence d'ions métalliques, d'oxygène et d'un agent réducteur, induisent des coupures dans des séquences d'acides nucléiques situées dans leur voisinage.

Le radical B est constitué par une base d'acide ribonucléique liée à la partie sucre du nucléOtide par une configuration anomérique alpha. Ce peut être l'adénine, la cytosine, la guanine ou l'uracile. Mais il est également possible d'utiliser des bases d'acides ribonucléiques modifiées, notamment halogénées ou azidées, par exemple la 5-bromo-uracile ou la 8-azidoadénine ; ou des dérivés aminés comme la 2-aminoadénine et ses dérivés substitués, par exemple sur le $N^6$ par un groupe aminoalkylène ou par un groupe azidophénylalkylène ; ou la guanine substituée sur le $O^6$, par exemple par un groupe (ω-alkylène)-9-acridine ou la 8-(ω-aminoalkyl)amino-adénine et ses dérivés substitués sur le $NH_2$ en ω par un groupe acridine.

Selon la présente invention, peuvent donc être considérées les bases usuelles, ainsi que la possibilité d'introduction de bases modifiées. Des "bases effectrices", susceptibles de se lier par covalence à un brin β complémentaire, pourront être introduites. Ainsi, la fonctionnalisation de C par un groupement aziridine en position 4 conduit à la formation de pontages covalents $CH_2$-$CH_2$ entre les deux brins complémentaires sur G.

Donc, plus particulièrement, le radical B est choisi parmi l'adénine, la cytosine, guanine, la 4-azido-cytosine et la 8-azido-adénine, l'uracile, la 5 bromo-uracile.
le radical -alk- est de préférence un radical alcoylène droit ou ramifié ayant de 1 à 10 atomes de carbone.

En particulier, à partir des fonctions alcool terminales 3′ ou 5′ de l'oligomère alpha, l'effecteur peut donc être introduit via une chaîne $(CH_2)_n$ liée à une fonctionnalisation Z′ de natures diverses à la partie osidique de l'oligomère.

A partir de la formule suivante :

3'

ou $-O-Z_1-(CH_2)_n-Z$

5'

on obtiendra, selon ce que représente $Z_1$, par exemple

. un phosphate ou méthyl phosphonate de formule

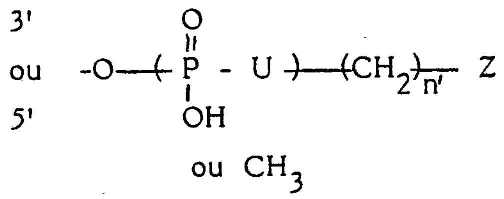

avec U = O, N ou S

. un éther de formule

3'

ou $-O-(CH_2)-(CH_2)_{n'}-Z$

5'

. un ester de formule

$-O-(CO)-(CH_2)_{n'}-Z$

ou encore

. un carbamate de formule générale

$-O-(CO\ NH)-(CH_2)_{n'}-Z$

La présente invention concerne également la synthèse des composés précédents sous forme de sel avec des bases ou des acides, et les composés sous forme racémique, ou sous forme isomères R ou S optiques purifiés ou en mélange, de formule générale précédente.

La présente invention a également pour objet les composés obtenus par le procédé selon l'invention, en particulier, les composés qui ne comportent pas uniquement les bases U et C.

Enfin, la présente invention a également pour objet des composés utiles notamment dans le procédé selon l'invention.

Ces composés sont les monomères ribonucléosides alpha substitués par un groupe phosphoramidite en position 3' dont la fonction hydroxyle en 2' est protégée par un groupe, tel que le groupe Ctmp ou le groupe TBDMS.

Plus particulièrement, ces composés sont les monomères répondant à la formule suivante

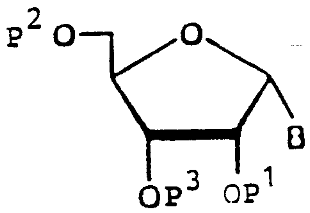

(II)

formule dans laquelle

$P^1$ = un groupe protecteur de la fonction hydroxyle tel que Ctmp, TBDMS

$P^2$ = H, ou un groupe protecteur de la fonction hydroxyle tel que Dmtr

$P^3$ = un groupe phosphoramidite tel qu'un groupe N,N dialkylamino phosphoramidite de formule (I) :

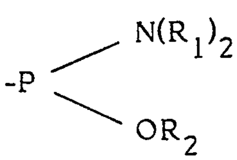

(I)

avec $R_1$ qui représente un alkyle en $C_1$ à $C_7$ éventuellement substitué identique ou différent, tel que $CH_3$, $(CH_3)_2CH$,

$R_2$ qui représente un alkyle en $C_1$ à $C_7$ éventuellement substitué tel que $CH_3$, $CH_2CH_2CN$

B = une base d'acide ribonucléique en configuration anomérique alpha par rapport au cycle glycosidique, base choisie parmi U, A, C, G et lesdites bases modifiées et/ou protégées.

La présente invention a également pour objet des composés ribonucléosides alpha immobilisés ou destinés à être immobilisés sur un support solide par l'intermédiaire de leur fonction hydroxyle en 2′ ou 3′.

On peut citer comme composés ribonucléosides alpha immobilisés ou destinés à être immobilisés sur le support solide, objets de l'invention, les composés répondant à la formule générale (III)

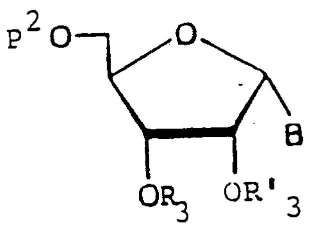

(III)

formule dans laquelle
B et $P^2$ ont les significations données précédemment
- l'un de $R_3$ et $R'_3$ représente H ou un groupe protecteur de la fonction hydroxyle tel que le groupe benzoyle,
- l'autre de $R_3$ et $R'_3$, un groupe fonctionnel permettant la fixation à un support solide par l'intermédiaire d'une fonction terminale, ou ledit groupe fonctionnel lié au support solide.

On peut citer comme groupe fonctionnel permettant la fixation ou étant lié sur support solide dôté d'un groupe amino, le groupe de formule

$$-\underset{\underset{O}{\|}}{C}-(CH_2)_p-\underset{\underset{O}{\|}}{C}R_4 \quad \text{(IV)}$$

avec p = 1 à 5
dans lequel $R_4$ représente OH, OH activé par un groupe d'activation tel que $C_6Cl_5$ ou un radical NH lié à un support solide tel que le radical NH d'une chaîne alkylamine fixée sur polymère, gel de silice ou billes de verre poreuses.

Les schémas 1 et 2 ci-après illustrent le procédé de préparation des monomères ribonucléosides alpha protégés substitués par des groupes phosphoramidites en 3′ avec respectivement le groupe protecteur Ctmp et le groupe protecteur TBDMS en 2′.

Dans ces schémas, B représente une base d'acide ribonucléique éventuellement modifiée et/ou protégée.

Ainsi, les guanine, cytidine et adénine alpha sont mis en oeuvre avec une protection benzoyle en position 2, 4 et 6 respectivement de la base.

SCHEMA 1

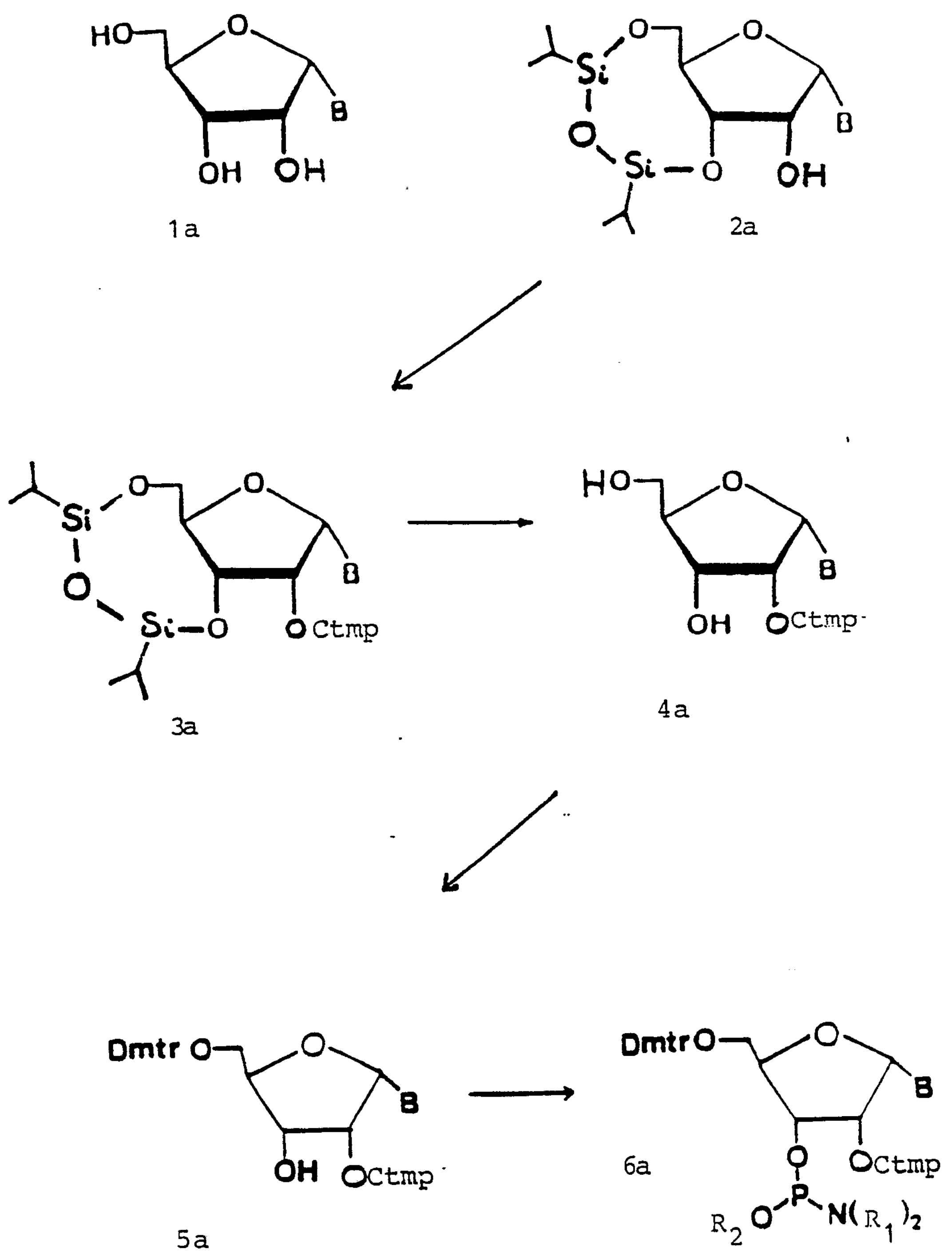

1a 2a 3a 4a 5a 6a

SCHEMA 2

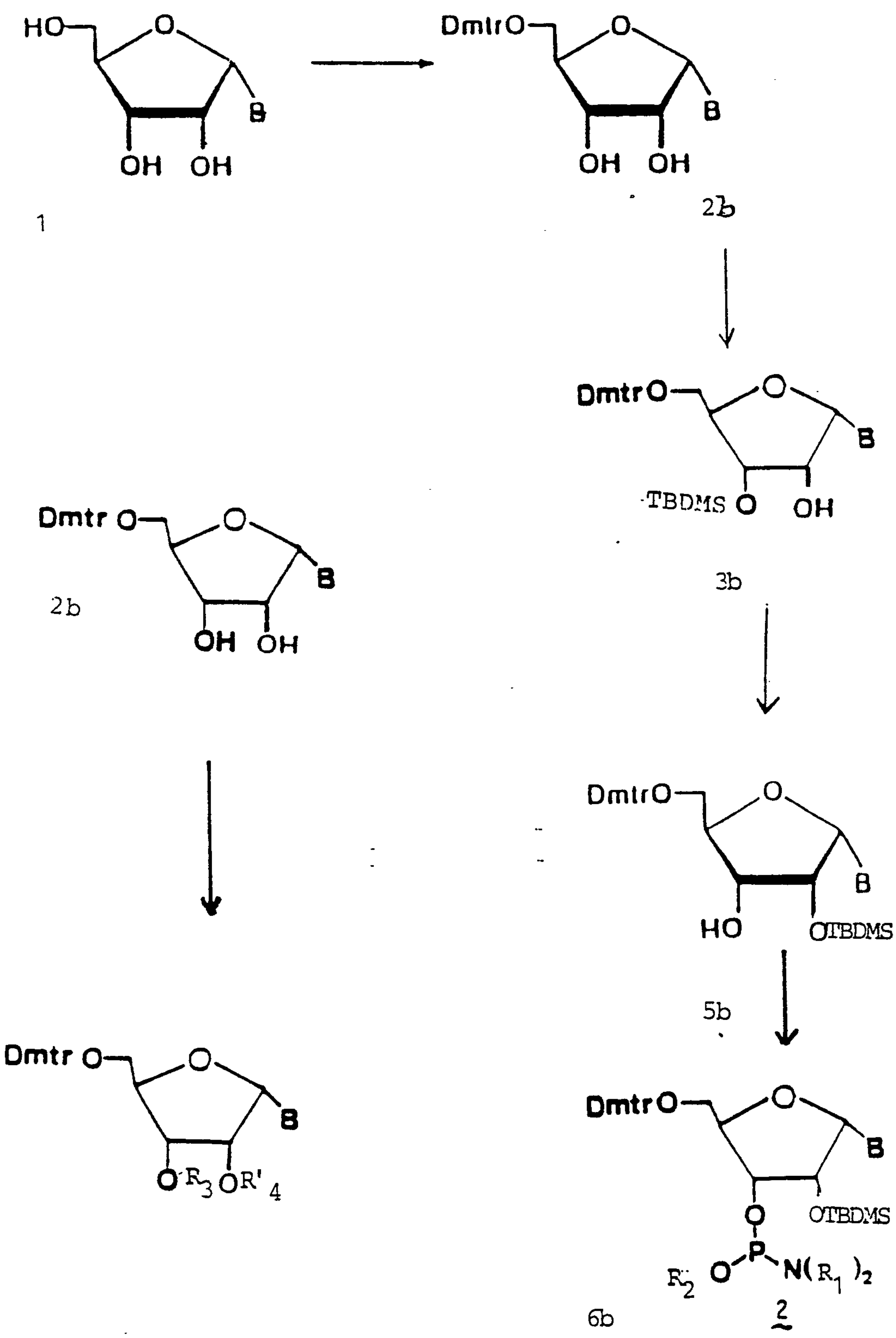

1

2b

3b

2b

5b

6b

2

1. Préparation des monomères (5a) (voir schéma 1 - $P^1$ = Ctmp)

Ils sont préparés à partir des ribonucléosides alpha (1a) obtenus selon des méthodes décrites dans la littérature. L'introduction sélective du groupement protecteur acido-labile Ctmp est effectuée selon la méthode suivante. Après traitement du composé (1a) par le tétraisopropyl-1,1,3,3 dichloro-1,3 disiloxane dans un mélange diméthylformamide-pyridine, le dérivé obtenu (2a) est traité par la [(chloro-2 méthyl-4) phényl]-1 méthoxy-4 tétrahydro-1, 2, 5, 6 pyridine en présence d'acide trifluoroacétique pour donner le composé (3a) dans du dioxane anhydre. Après purification, ce composé est soumis à un traitement par une solution de $Et_4$ NBr et KF dans du $CH_3CN$ à 50°C pendant 1 heure. La protection tétraisopropyl-1,1,3,3 disilyle est ainsi sélectivement ôtée et on obtient le composé (4a).

Le dérivé nucléosidique (5a) est obtenu après tritylation du composé (4a) par le chlorure de diméthoxy-4,4′ trityle en milieu pyridine.

2. Préparation des synthons nucléosidiques 5b (voir schéma 2) ($P^1$ = TBDMS)

Les dérivés nucléosidiques (2b) sont obtenus après protection, par exemple après tritylation des dérivés (1b) correspondant par le chlorure de diméthoxy -4,4′ trityle en milieu pyridine.

On obtient ensuite le dérivé (3b) substitué en position 3′ après réaction avec le chlorure de terbutyldiméthylsilyl en présence d'imidazole dans de la pyridine anhydre.

Les dérivés (5b) sont enfin obtenus à partir des dérivés (3b) par migration intramoléculaire du groupement TBDMS en présence de triéthylamine dans du méthanol.

3. Préparation des ribonucléoside-phosphoramidites alpha (6a-b) (voir schéma 1 et 2 respectivement)

Les ribonucléoside-phosphoramidites alpha N- protégés (6a-b) ont été obtenus à partir des O-diméthoxytrityl-5′ ribonucléosides alpha N-protégés correspondants (5a-b). Le dérivé nucléosidique (5a) a été traité par de la chloro N,N-di-isopropylaminométhoxyphosphine en présence de N,N,N-diisopropyléthylamine (DIEA) dans le dichlorométhane, sous atmosphère inerte, pour donner le dérivé 6a pour lequel $R_2 = CH_3$.

Le dérivé nucléosidique (5b) a été traité par le N,N,N′,N′-tétraisopropyl-phosphorodiamidite de 2-cyanoéthyle (1,1 équivalent molaire) en présence de tétrazolure de diisopropylammonium (0,5 équivalent molaire) dans le dichlorométhane anhydre, pour donner le dérivé 6b pour lequel $R_2 = CH_2\text{-}CH_2\text{-}CN$.

4. Préparation des synthons nucléosidiques 7 constituant les extrémités 2′ ou 3′ des oligoribonucléotides fixés sur le support solide (voir schéma 2)

Les synthons nucléosidiques 7, constituant l'extrémité 3′ des oligonucléotides sont obtenus en deux étapes à partir des dérivés 1. Ces deux étapes sont :

a) la tritylation sélective de l'hydroxyle-5′ des dérivés 1 par le chlorure de diméthoxy-4,4′ trityle pour donner les dérivés 2b, puis

b) la benzoylation des hydroxyles en position 2′ ou 3′ des dérivés 2b par le chlorure de benzoyle.

5. Greffage des synthons 7 sur support solide (schéma 3)

Ce greffage est illustré sur le schéma 3. On fait réagir une solution de mélange isomérique monobenzoylé-2′ (ou 3′) (7), d'anhydride succinique et de diméthylaminopyridine (DMAP) dans la pyridine. On obtient le composé (8) comportant un groupe succinyle en position 2′ ou 3′ l'autre hydroxyle vicinal étant protégé par un groupe benzoyle. Le dérivé (8) est ensuite transformé en dérivé pentachlorophényle succinate 3′ ou 2′ lequel est ensuite mis en réaction avec le groupement amino d'une longue chaîne alkylamine fixée par exemple sur billes de verre poreuses (LCA-CPG) pour donner le composé (10) fixé sur le support solide.

SCHEMA 3

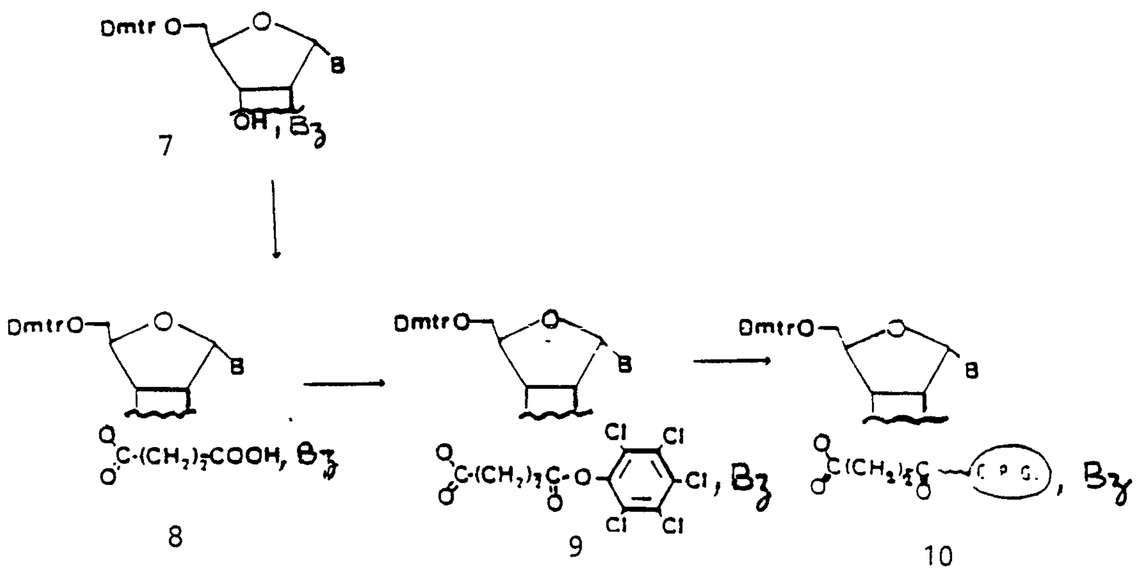

7

8

9

10

6. Assemblage des oligoribonucléotides alpha entièrement protégés

Le composé (10) lié de façon covalente au support LCA-CPG est introduit dans un petit reacteur. L'élongation de l'oligorigonucléotide alpha est effectuée par une succession de cycles de synthèse.

Outre les lavages, chaque cycle comprend une détritylation, une condensation, une acétylation et une oxydation. Au terme du nombre de cycles requis, le support est traité par mélange thiophénol/triéthylamine/dioxane (1/2/3, v/v/v) pendant une heure pour enlever le groupement méthyle ou cyanoéthyle des phosphates. Puis le support est traité avec de l'ammoniaque concentré afin d'enlever les groupement protecteurs des amines hétérocycliques et détacher les produits nucléotidiques du support solide. L'oligoribonucléotide désiré, portant encore le groupement diméthoxytrityl à son extrémité 5′ est purifié par chromatographie liquide haute performance sur colonne. Un traitement par de l'acide acétique permet de déprotéger son extrémité 5′.

De tels cycles de synthèses sont bien connus dans les techniques touchant à la synthèse des oligonucléotides.

D'autres caractéristiques et avantages de la présente invention apparaîtront à la lumière des exemples qui vont suivre.

**EXEMPLE 1 Préparation du synthon phosphoramidite entièrement protégé de l'uridine alpha**

a) Protection de l'uridine alpha

a.1) Protection du 5′OH du ribose par le groupement acido-labile diméthoxy-4,4′ trityle (Dmtr)

a.2) Protection du 2′OH du sucre

L'utilisation de 2 groupements protecteurs a été envisagée :

Le groupement acido-labile 1-[(2-chloro-4-méthyl)phényl]-4-méthoxypiperidin-4-yl (ou Ctmp) proposé dans un autre contexte proposé par C.B. Reese Nucléosides, Nucléotides, N° 6, P 121, 1987.

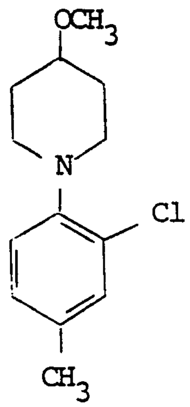

Ce groupement présente l'intérêt d'être compatible avec l'utilisation du Dmtr car il reste intact dans les conditions de déprotection de celui-ci, contrairement au groupement 4-méthoxytétrahydropyrane-4-yl (Mthp). Cependant, la synthèse du réactif selon C.B. Reese Tet. Letters. Vol 27, N° 20, P 2291 (1986) nécessite de nombreuses étapes aux rendements très moyens. On a donc envisagé l'utilisation d'un autre groupement protecteur.

Le groupement tert-butyldiméthylsilyl (TBDMS) proposé dans une autre application par K.K. Ogilvie et al. Can. J. Chem. 57, 2230 (1979). Celui-ci est éliminé par un traitement aux ions $F^-$ c'est-à-dire par le fluorure de tétrabutylammonium (TBAF) dans le THF anhydre.

b) Synthèse de l'uridine alpha entièrement protégée pour l'élongation de l'oligoribonucléotide alpha

b.1. Utilisation du groupement protecteur Ctmp pour le 2'OH du sucre

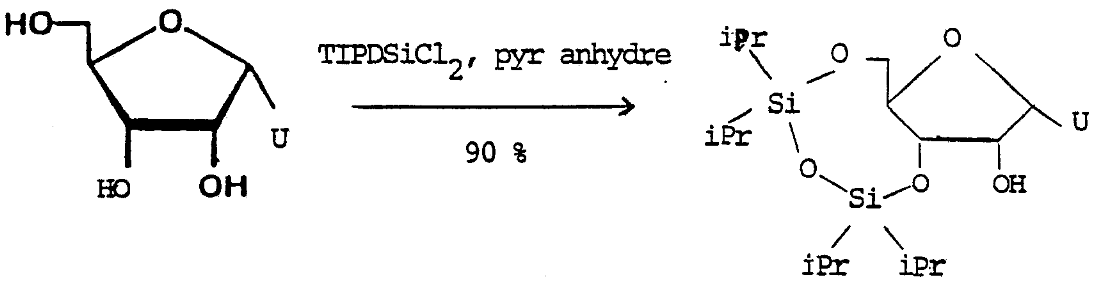


A une solution de α-uridine[1] (1,831 g, 7,5 mmoles) dans de la pyridine anhydre (15 ml) on ajoute du dichloro-1,3 tétraisopropyl-1,1,3,3 disiloxane[2] (2,5 ml, 7,9 mmoles). La solution est agitée à température ambiante pendant 2 heures puis est concentrée de moitié par évaporation sous vide. Le résidu est versé dans une solution aqueuse saturée de bicar bonate de sodium (20 ml) et le mélange est extrait avec du dichlorométhane (40 ml et 2 × 20 ml). Les phases organiques rassemblées sont séchées sur sulfate de sodium et filtrées. Le filtrat est évaporé sous pression réduite puis coévaporé avec du toluène (3 × 20 ml). Le résidu est chromatographié sur colonne de gel de silice et le produit désiré est élué avec un mélange de méthanol (de 0 à 10%) et de dichlorométhane. Les fractions contenant le produit désiré pur sont rassemblées et évaporées sous pression réduite. Le résidu est redissous dans du benzène puis lyophilisé. On obtient 3,3 g d'une poudre blanche. Rendement 90%.

RMN $^1H$ ($CDCl_3$) δ 9,03 (s, 1H, NH) ; 7,46 (d, 1H, $H_6$, $J_{5-6}$ 8,15 Hz) ; 6,14 (d, 1H, $H_{1'}$, $J_{1'-2'}$ 2,76 Hz) ; 5,69 (d, 1H, $H_5$) ; 4,47 (m, 2H, $H_{2'}$ et $H_{3'}$) ; 4,17 (m, 1H, $H_{4'}$) ; 4,03 (m, 2H, $H_{5'}$ et $H_{5''}$) ; 3,13 (s, 1H, $OH_{2'}$) ; 1,06 (m, 28H, CH et $CH_3$ du groupement TIPS).

Références : [1] - D.H. Shannahoff et R.A. Sanchez, *J. Org. Chem.* 38, 593 (1973).
[2] - W.T. Markiewicz, *J. Chem. Res., Synop.,* 24 (1979) ; *J. Chem. Res.*, 181 (1979.

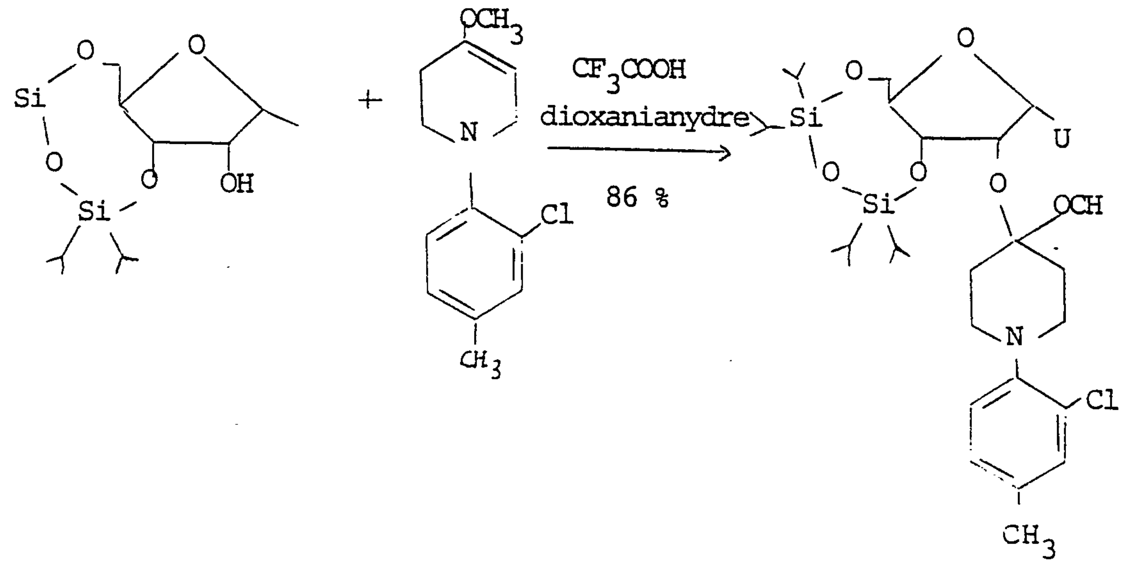


Dans un ballon contenant une solution de [chloro-2 méthyl-4)phényl]-1 méthoxy-4 tétrahydro-1,2,5,6 pyridine[1] (3,46 g, 14,54 mmoles) et de 0-(tétraisopropyl-1,1,3,3 disiloxane diyl-1,3)-3′,5′ α-uridine (0,782 g, 1,615 mmoles) dans du dioxanne anhydre (8 ml) on ajoute 0,55 ml d'acide trifluoroacétique. Le mélange est agité pendant 5 heures à température ambiante puis est neutralisé par addition de triéthylamine (1,5 ml). Le mélange est immédiatement évaporée à sec sous pression réduite et le résidu huileux est chromatographié sur une colonne de gel de silice. L'élution est effectuée avec des proportions croissantes d'acétone (0 à 10%) dans du dichlorométhane. Les fractions contenant le produit désiré pur sont rassemblées et évaporées à sec sous pression réduite. On obtient une mousse jaune-pâle (1 g, 85,5%).

RMN $^1H$ ($CDCl_3$), δ 8,53 (d, 1H, NH, $J_{NH-H_5}$ 2,25 Hz) ; 7,52 (d, 1H, $H_6$, $J_{5,6}$ 8,17 Hz) ; 6,86-7,16 (m, 3H, H aromatiques du groupement Ctmp) ; 6,26 (d, 1H, $H_{1'}$, $J_{1'-2'}$ 3,63 Hz) ; 5,75 (dd, 1H, $H_5$) ; 4,62 (pseudo t, 1H, $H_{2'}$) ; 4,42 (m, 1H, $H_{3'}$) ; 3,9-4,15 (m, 3H, $H_{4'}$, $H_{5'}$ et $H_{5''}$) ; 3,17 (s, 3H, $OCH_3$) ; 1,55-3,1 (m, 8H, $CH_2$ du pyridinyl) ; 2,26 (s, 3H, $CH_3$ du toluyl) ; 1,1 (m, 28H, CH et $CH_3$ du groupement TIPS).

Références : [1] - C.B. Reese, H.T. Serafinowska et G. Zappia, *Tet. Lett.*, 27, 2291 (1986).

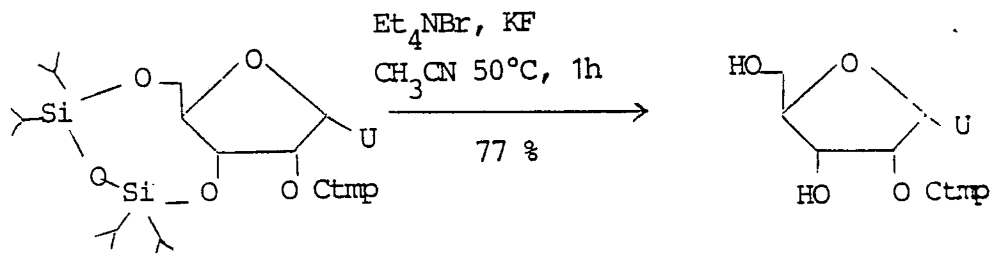


A une solution du composé précédemment obtenu (1 g, 1,38 mmoles) dans un mélange d'acétonitrile (41,5 ml) et d'eau (0,41 ml), on ajoute du bromure de tétraéthylammonium (1,74 g, 8,28 mmoles) et du fluorure de potassium[1] (0,481 g, 8,28 mmoles). Le mélange est agité vigoureusement à 50°C pendant 1 heure puis est filtré. Le filtrat est évaporé à sec sous pression réduite et le résidu est redissous dans du dichlorométhane (50 ml). Cette solution organique est lavée avec de l'eau (3 × 30 ml) puis est séchée sur sulfate de sodium et évaporée à sec. Le résidu est chromatographié sur une colonne de gel de silice. L'élution est effectuée avec des mélanges de méthanol (0,5 à 10%) et de dichlorométhane. Les fractions contenant le produit désiré pur sont rassemblées et évaporées à sec. On obtient un résidu mousseux incolore (0,51 g, 77%).

RMN $^1H$ ($CDCl_3$) δ 8,85 (s, 1H, NH) ; 7,75 (d, 1H, $H_6$, $J_{5-6}$ 8,18 Hz) ; 6,88-7,15 (m, 3H, H aromatiques du groupement Ctmp) ; 6,48 (d, 1H, $H_{1'}$, $J_{1'-2'}$ 6,82 Hz) ; 5,71 (d, 1H, $H_5$) ; 4,86 (pseudo t, 1H, $H_{2'}$) ; 4,40 (m, 1H, $H_{3'}$) ; 4,24 (m, 1H, $H_{4'}$) ; 3,6-3,9 (m, 2H, $H_{5'}$ et $H_{5''}$) ; 3,27 (s, 3H, OC $H_3$) ; 3,03 (m, 1H, $OH_{3'}$) ; 2,75-3,1 (m, 4H, $2CH_2$ du groupement Ctmp) ; 2,33 (m, 1H, $OH_{5'}$) ; 2,26 (s, 3H, $CH_3$ du toluyl) ; 1,75-2,15 (m, 4H, $2CH_2$ du groupement Ctmp).

Références : [1] - T. Kamimura, M. Tsuchiyan K. Urakami, K. Koura, M. Sekine, K. Shinozaki, K. Miura et T. Hata, *J. Amer. Chem. Soc.*, 106, 4552 (1984).

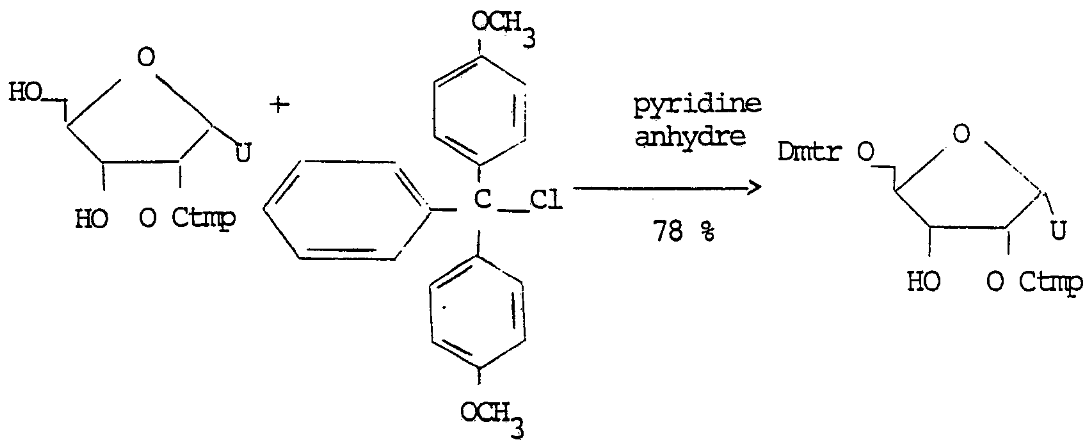


(Dmtr-Cl)

A une solution du composé précédement obtenu (0,51 g, 1,06 mmoles) dans la pyridine anhydre (5 ml) on ajoute du chlorure de diméthoxytrityle (0,430 g, 1,27 mmoles). Le mélange est agité pendant 1,5 heure à température ambiante puis on ajoute du méthanol (2 ml). Après 5 minutes d'agitation, le mélange réactionnel est versé sur une solution aqueuse saturée de bicarbonate de sodium (30 ml) et les produits sont extraits avec du dichlorométhane (3 × 30 ml). Les phases organiques rassemblées sont séchées sur sulfate de sodium et filtrées. Le filtrat est évaporé à sec sous pression réduite et le résidu est chromatographié sur une colonne de gel de silice. L'élution est effectuée avec un mélange de méthanol (0 à 10%) dans le dichlorométhane. Les fractions contenant le produit désiré pur sont regroupées et évaporées à sec. Le résidu est précipité dans de l'éther de pétrole. On obtient, après séchage, une poudre incolore (0,645 g, 78%).
RMN $^1$H ($CDCl_3$ δ 8,3 (d, 1H, NH, $J_{NH-H_5}$ 2,23 Hz) ; 7,74 (d, 1H, $H_6$, $J_{5-6}$ 8,22 Hz) ; 6,75-7,5 (m,16H, aromatiques) ; 6,6 (d, 1H, $H_{1'}$, $J_{1'-2'}$ 7,07 Hz) ; 5,69 (dd, 1H, $H_5$) ; 5,15 (m, 1H, $H_{2'}$) ; 4,46 (m, 1H, $H_{3'}$) ; 4,10 (m, 1H, $H_{4'}$) ; 3,79 (s, 6H, $OCH_3$ du groupement Dmtr) ; 3,30 (s, 3H, $OCH_3$ du groupement Ctmp) ; 3,05-3,50 (m, 2H, $H_{5'}$ et $H_{5''}$) ; 2,86-3,05 (m, 4H, $2CH_2$ du groupement Ctmp) ; 2,78 (s, 1H, $OH_{3'}$) ; 2,27 (s, 3H, $CH_3$ du groupement Ctmp) ; 1,7-2,05 (m, 4H, $2CH_2$ du groupement Ctmp).

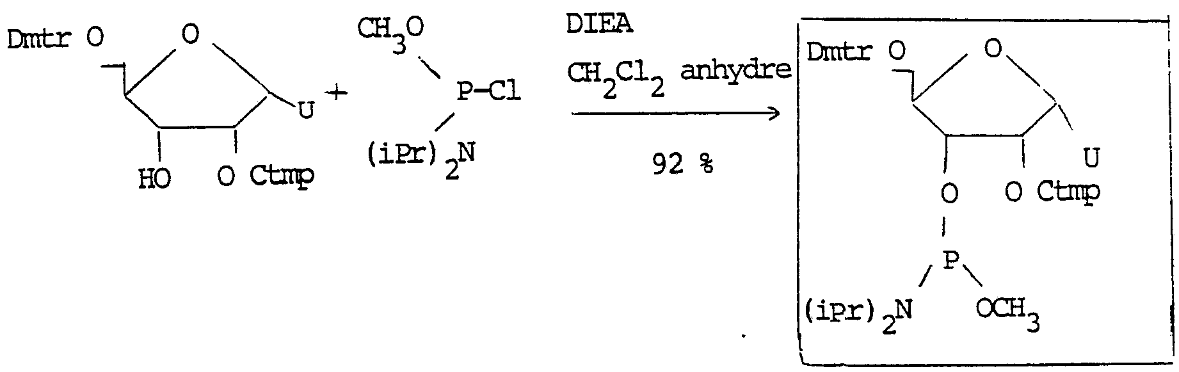


A une solution du composé précédemment obtenu (1,853 g, 2,36 mmoles) dans du dichlorométhane anhydre (7 ml) on ajoute de la N,N,N-diisopropyléthylamine (1,65 ml, 9,44 mmoles) et de la chloro N,N-diisopropylaminométhoxyphosphine (1,15 ml, 5,9 mmoles). Le mélange est agité pendant 30 minutes à température ambiante, sous atmosphère d'argon, puis est versé dans une ampoule à décanter contenant de l'acétate d'éthyle (80 ml). La solution résultante est lavée avec de la saumure (4 × 50 ml). La phase organique est séchée sur sulfate de sodium, filtrée et évaporée à sec sous pression réduite. Le résidu est chromatographié sur colonne de gel de silice et l'élution a lieu avec des mélanges de cyclohexane, de dichlorométhane et de triéthylamine (99/0/1 à 0/99/1, v/v/v). Les fractions contenant le produit désiré pur sont rassemblées, évaporées à sec et le résidu redissous dans du benzène est soumis à lyophilisation. On obtient une poudre incolore (2,056 g, 92 %).
RMN $^{31}$P ($CD_3CN$) δ 158,52 et 155,78.

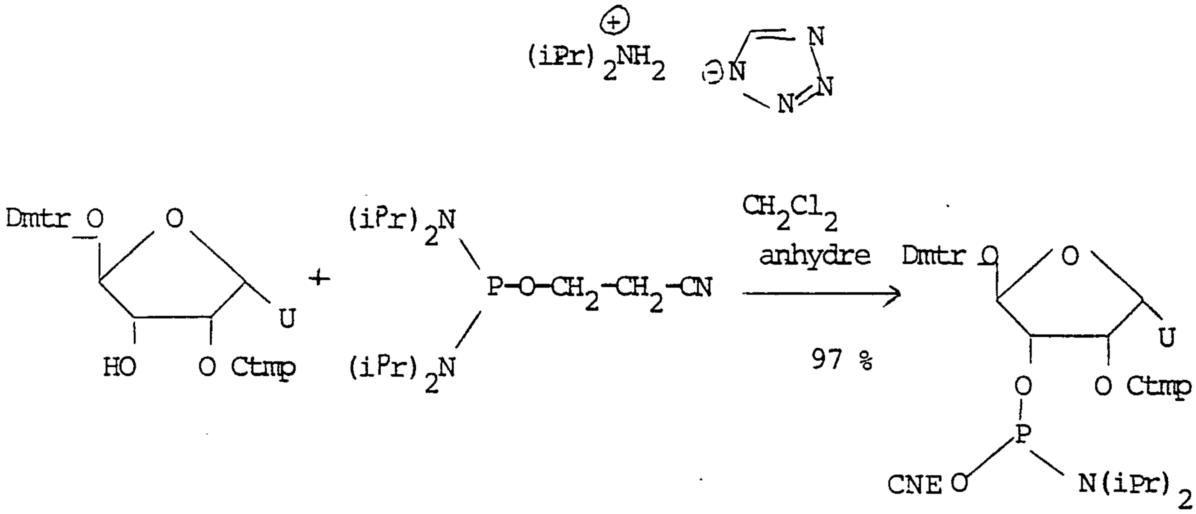


A un mélange de O-Dmtr-5′ -Ctmp-3′ α-uridine (0,644 g, 0,821 mmole) et de tétrazolure de diisopropylammonium (0,070 g, 0,41 mmole) est ajouté successivement une solution de N,N,N′,N′-tétra-iso-propylphosphorodiamidite de 2-cyanoéthyle[1] (0,272 g, 0,9 mmole) dans du dichlorométhane anhydre (1 ml) et du dichlorométhane anhydre (3,1 ml). La solution est agitée pendant 4 heures à température ambiante et une quantité supplémentaire de phosphoramidite est ajoutée (...). La solution est agitée pendant une période supplémentaire de 18 heures puis est chauffée à 50°C pendant 7 heures. Le mélange réactionnel est alors versé sur une solution aqueuse saturée de bicarbonate de sodium (10 ml). A ce mélange est ajouté de l'acétate d'éthyle (20 ml) et la phase organique est lavée avec de la saumure (3 × 10 ml), séchée sur sulfate de sodium et filtrée. Après évaporation des solvants, le résidu est chromatographié sur colonne de gel de silice, en utilisant le même éluant que précédemment. Après lyophylisation, le composé désiré est recueilli sous forme d'une poudre incolore (0,789 g, 97%).
RMN $^{31}P$ ($CD_3CN$) δ 152,35 et 149,68.
Références : [1] . J. Nielsen et O. Dahl, *Nucl. Acid. Res.*, 15, 3626 (1987).

b.2 Utilisation du groupement protecteur TBDMS pour le 2′OH du sucre

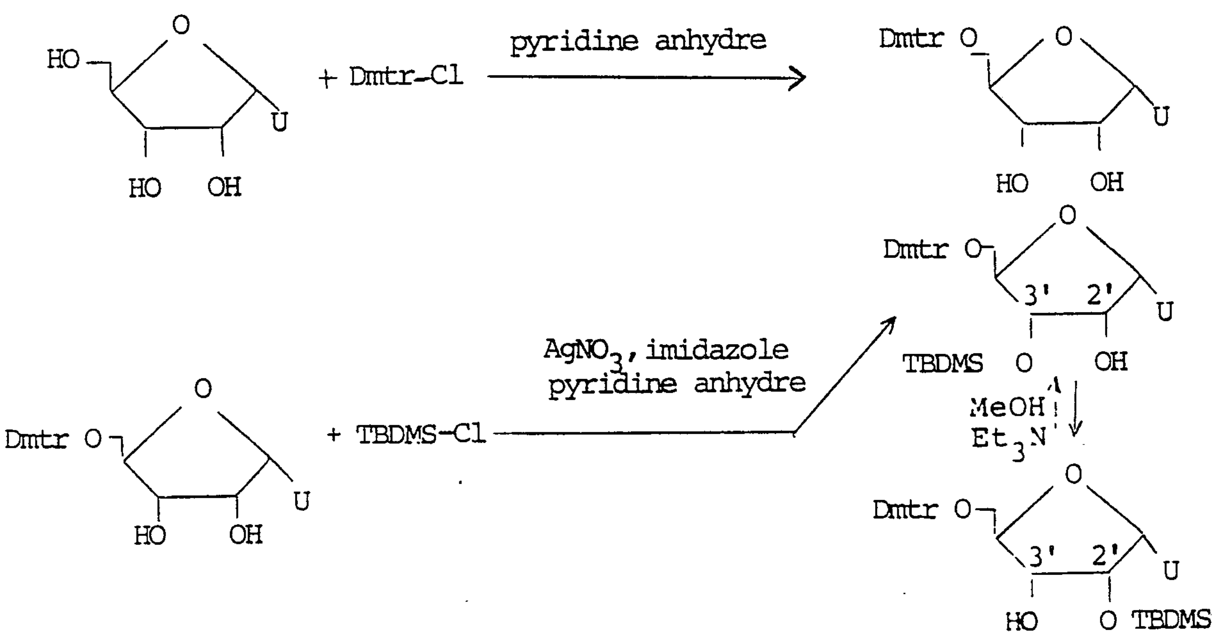


Réf. : K.K. Ogilvie, K.K. Schifman and C.L. Penny, Can. J. chem. 57, 2230 (1979)
S.S. Jones and C.B. Reese, J. Chem. Soc. Perkin I 1979 p 2762
K.K. Ogilvie, Carbohydrate Research, 89 (1981) 203-210
W.L. Sung, S.A. Narang, Can. J. Chem. 60, 111 (1982)

Dans des conditions identiques à celles décrites plus haut, l'action du chlorure de diméthoxytrityle sur de l'α-uridine a fourni la O-diméthoxytrityle-5 α-uridine avec un rendement de 73%. A une solution de ce composé (0,546 g, 1 mmole) dans du tétrahydrofuranne anhydre (10 ml) on ajoute successivement de la

pyridine (0,3 ml, 3,7 mmoles), du nitrate d'argent (0,204 g, 1,2 mmoles) et du chlorure de tertiobutyldiméthylsilyle (TBDMS-Cl, 0,196 g, 1,3 mmoles). Le mélange réactionnel est agité à température ambiante pendant 25 heures puis est filtré. Le filtrat est versé dans une ampoule à décanter contenant une solution aqueuse saturée de bicarbonate de sodium (10 ml). Les produits sont extraits avec du dichlorométhane (2 × 20 ml). Les phases organiques sont rassemblées et évaporées à sec sous pression réduite. Le résidu est chromatographié sur colonne de gel de silice en utilisant comme éluant un mélange de méthanol (0 à 5%) et de dichlorométhane). La 0-dimethoxytrityl-5′ 0-tertiobutyldiméthylsilyl-3′ α-uridine (0,514 g) est obtenue avec un rendement de 78%.

RMN $^1H$ ($CDCl_3$) δ 8,73 (s, 1H, NH) ; 7,55 (d, 1H, $H_6$, $J_{5-6}$ 8,17 Hz) ; 6,80-7,50 (m, 13H, H aromatiques) ; 6,34 (d, 1H, $H_{1'}$, $J_{1'-2'}$ = 4,38 Hz) ; 5,72 (d, 1H, $H_5$) ; 4,44 (m, 1H, $H_{2'}$) ; 4,35 (m, 1H, $H_{3'}$) ; 4,23 (m, 1H, $H_{4'}$) ; 3,80 (, 6H, $(OCH_3)_2$) ; 3,0-3,5 (m, 2H, $H_{5'}$ et $H_{5''}$) ; 2,74 (d, 1H, $OH_{2'}$) ; 0,82 (s, 9H, $(CH_3)_3C$) ; 0,02 et - 0,07 (2s, 6H, $Si(CH_3)_2$).

Ce dernier composé (0,435 g, 0,66 mmole) est dissous dans une solution de triéthylamine (0,009 ml) dans du méthanol (44 ml). Le mélange est agité à température ambiante pendant 24 heures puis les solvants sont évaporés sous pression réduite. Le résidu qui est un mélange des deux isomères TBDMS-3′ et TBDMS-2′ est chromatographié sur une colonne de gel de silice. Après élution par un mélange de méthanol(3 à 4%) et de dichlorométhane, les fractions contenant l'isomère substitué en 2′ sont rassemblées et évaporées à sec. Le résidu mis en solution dans du benzène est soumis à lyophilisation. On recueille une poudre incolore (0,095 g). Les fractions contenant l'isomère substitué en 3′ (isomère plus polaire) sont rassemblées et évaporées. Le résidu est soumis à nouveau à un traitement par une solution de triéthylamine dans le méthanol puis à une purification par chromatographie comme précédemment. Ainsi, on recueille en deux lots (0,204 g, 47%) de l'isomète O-diméthoxytrityl-5′ O-tertiobutyldiméthylsilyl-2′ α-uridine.

RMN $^1H$ ($CDCl_3$) δ 8,57 (s, 1H, NH) ; 7,59 (d, 1H, $H_6$, $J_{5-6}$ 8,15 Hz) ; 6,83-7,41 (m, 13H, H aromatiques) ; 6,49 (d, $H_{1'}$, $J_{1'-2'}$ 5,94 Hz) ; 5,69 (d, 1H, $H_5$) ; 4,85 (m, 1H, $H_{2'}$), 4,35 (m, 1H, $H_{4'}$), 4,07 (m, 1H, $H_{3'}$) ; 3,79 (s, 6H, $(OCH_3)_2$) ; 3,06-3,52 (m, 2H, $H_{5'}$ et $H_{5''}$) ; 2,54 (d, 1H, $OH_{3'}$) ; 0,85 (s, 9H, $(CH_3)_2$) ; O,06 et -0,13 (2s, 6H, $Si(CH_3)_2$).

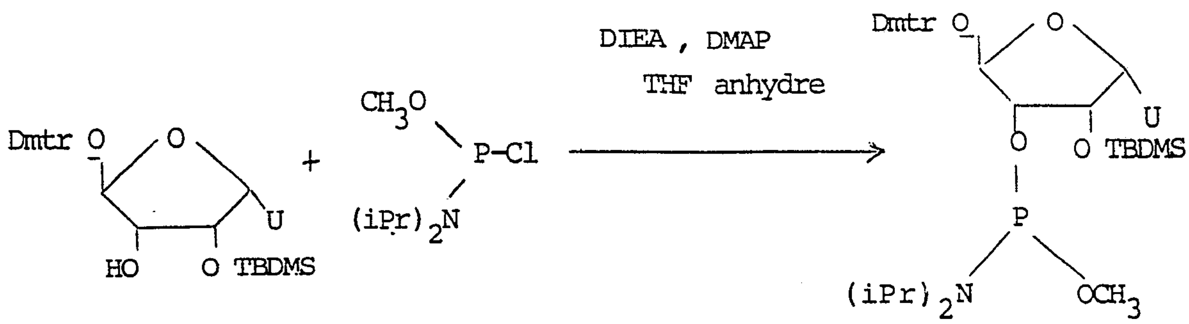


RMN$^{31}$p δ = 152,65 et 151,58

**EXEMPLE 2 Préparation du synthon uridine alpha 3′ terminal (fixé sur le support LCA-CPG)**

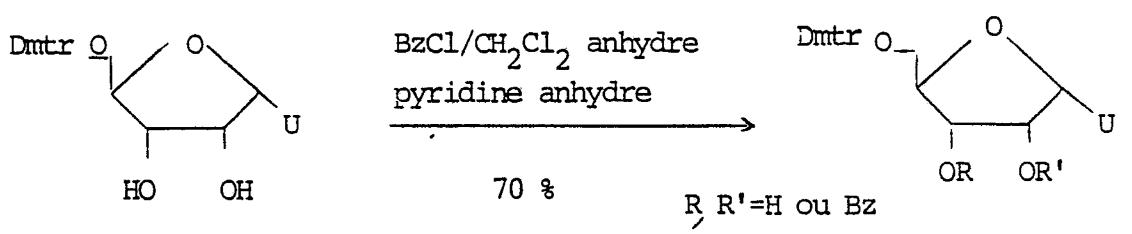


Une solution de O-diméthoxytrityl-5′ α-uridine (0,796 g, 1,46 mmoles) dans de la pyridine anhydre (7,3 ml), gardée sous atmosphère d'argon, est refroidie à - 45° C. On y ajoute, goutte à goutte et par l'intermédiaire d'une seringue, une solution de chlorure de benzoyle[1] (0,186 ml 1,61 mmoles) dans du dichlorométhane anhydre (0,58 ml). L'addition dure 5 minutes et le mélange réactionnel est conservé à une température de -45° C pendant 30 mn puis à une température de -20° C pendant 3 heures supplémentaires. De l'eau (0,2 ml) est ajoutée au mélange réactionnel qui est ensuite versé dans une ampoule à décanter contenant une solution aqueuse saturée de bicarbonate de sodium (35 ml). Les produits sont extraits avec du dichlorométhane (30 ml et 2 × 15 ml). Les phases organiques sont rassemblées, lavées avec de l'eau (20 ml) et évaporées à sec. Le résidu est chromatographié sur une colonne de gel de silice. L'élution a lieu avec un mélange d'acétone (0 à 15%) dans le dichlorométhane. Les fractions contenant le mélange de 0-diméthoxytrityl-5′ O-benzoyl 2′ (ou 3′) α-uridine sont rassemblées et évaporées à sec. Le résidu est précipité dans de l'éther de pétrole pour donner une poudre incolore (0,652 g, 70%).

Références : [1] . T. Kempe, F. Chow, W.I. Sundquist, T.J. Nardi, B. Poulson et S.M. Peterson, *Nucl. Acid Res.*, 10, 6695 (1982).

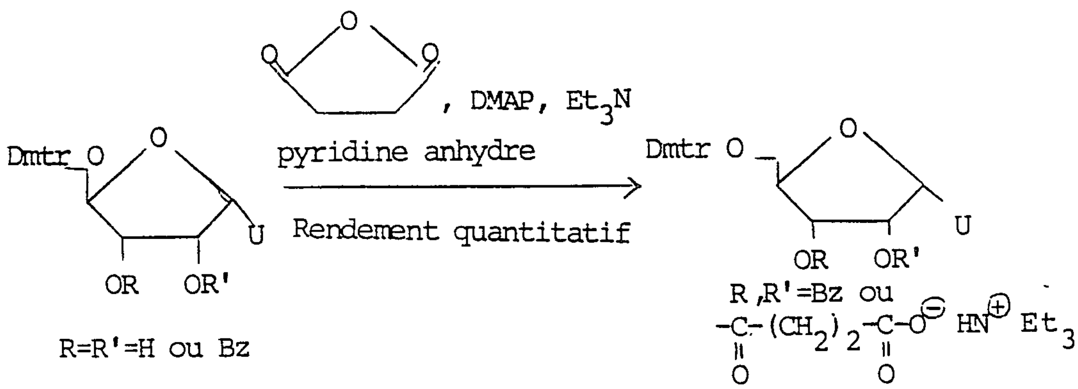


La O-diméthoxytrityl-5′ O-benzoyl-2′(ou 3′) O-succinyl-3′(ou 2′) α-uridine a été obtenue à partir du composé précédemment décrit selon un procédé identique à celui décrit par T. Tanaka et al.[1] .
Rendement quantitatif.

Références : [1] . T; Tanaka, S. Tamatsukuri et M? Ikehara, *Nucl. Acid. Res.*, 14, 6265 (1986).

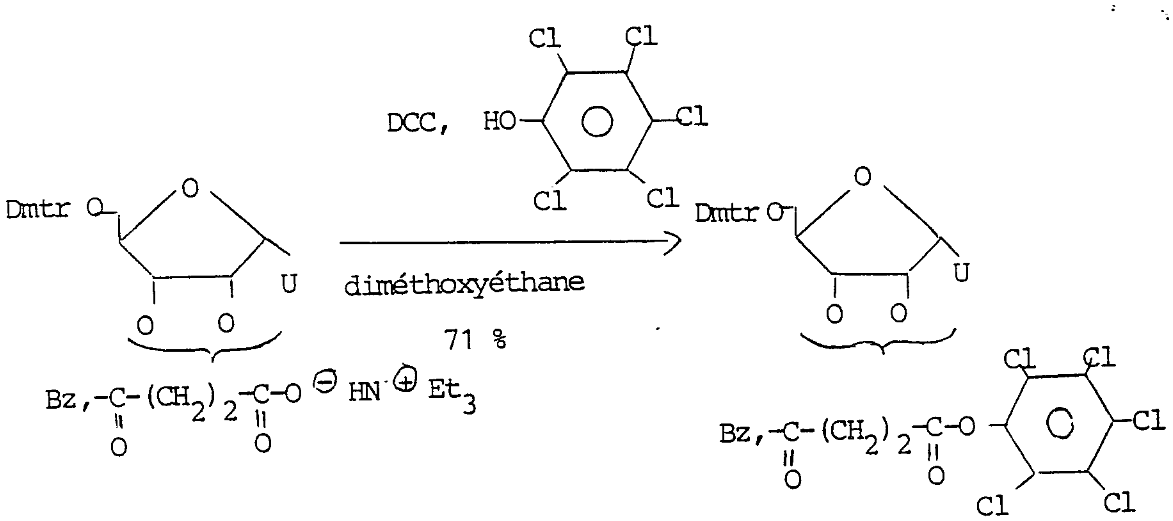


L'ester de pentachlorophényle correspondant au succinate précédemment reporté a été obtenu par condensation en présence de dicyclohexylcarbodiimide, selon un procédé identique à celui décrit par Gough et al.[1] .
Rendement 71%.

Références : [1] . G.R. Gough, M.J. Brunderer et P.T. Gilham, *Tetrahedron Lett.*, 22, 4177 (1981).

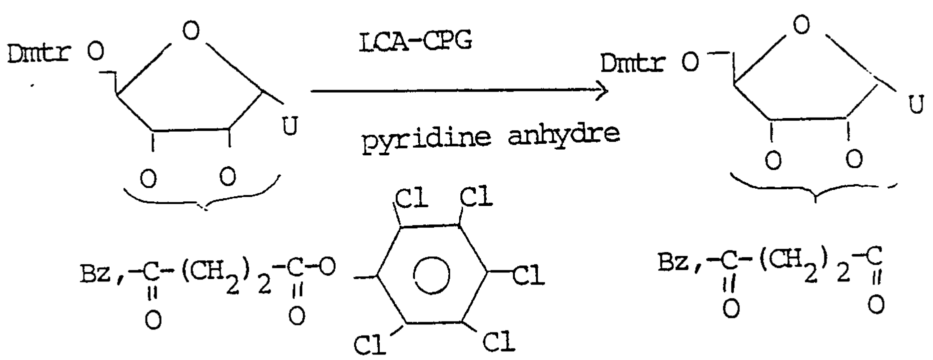


Le support "long chain alkylamine controlled pore glass" (Pierce) (0,5 g) est activé avec de la triéthylamine (0,03 ml) dans de la pyridine anhydre (2 ml). Après évaporation des solvants, le résidu est mis en suspension dans une solution de l'ester actif précédemment reporté (0,152 g, 0,15 mmole) dans de la pyridine anhydre (1,5

ml). Le mélange est agité pendant 72 heures à température ambiante puis les billes de verre sont essorées, lavées avec de la pyridine (2 ml) et du dichlorométhane (3 × 2 ml) et séchées sous vide. Ensuite, ces billes de verre sont mises en suspension dans une solution (2,4 ml) préparée à partir d'un mélange de N,N-diméthylamino-4 pyridine (180 mg) dans du tétrahydrofuranne anhydre (2 ml), d'anhydride acétique (0,0187 ml, 0,198 mmole), de lutidine (0,0198 ml, 0,171 mmole) et du tétrahydrofuranne (1 ml). Le mélange est agité pendant 10 minutes et le matériau insoluble est essoré, lavé avec du tétrahydrofuranne (2 × 2 ml), du dichlorométhane (4 × 2 ml), et séché sous vide. Le dosage spectrophotométrique du cation diméthoxytrityle, libéré par traitement (à l'aide d'une solution décimolaire d'acide paratoluènesulfonique dans l'acétonitrile) d'un échantillon de billes de verre ainsi fonctionnalisées, indique une fonctionnalisation de 26 μmoles par gramme.

**EXEMPLE 3 Préparation du synthon phosphoroamidite entièrement protégé de N-benzoyl-4 alpha-cytidine**

**Synthèse de l'α-cytidine.**

**1) Synthèse de (tri-O-acétyl 2′,3′,5′ α-D-ribofurannosyi)-1 Uracile.**

A une solution de α-uridine[1] (14,77 g, 60,48 mmoles) dans de la pyridine anhydre (10,5 ml) on ajoute de l'anhydride acétique (104 ml). La solution est agitée à température ambiante pendant 2,5 heures. Après l'hydrolyse de l'anhydride acétique en excès par du méthanol (200 ml) ajouté avec précaution, le mélange est évaporé à sec sous pression réduite. Le résidu huileux de couleur orangée, obtenu est dissous dans de l'eau (200 ml) puis neutralisé par une solution aqueuse saturée de bicarbonate de sodium. Le mélange est extrait avec du dichlorométhane (4 × 200 ml). Les phases organiques rassemblées sont séchées sur sulfate de sodium et filtrées. Le filtrat est évaporé sous pression réduite puis coéavporé avec du toluène. On obtient une mousse blanche (24,3 g, 57,5 mmoles). Rendement 95%.

RMN $^1$H ($CDCl_3$) δ 8,99 (s, 1H, NH) ; 7,44 (d, 1H, $H_6$, $J_{5-6}$ 8,2 Hz) ; 6,35 (d, 1H, $H_{1'}$, $J_{1'-2'}$ 4,75 Hz) ; 5,73 (d,1H, $H_5$) ; 5,69 (pseudo t, 1H, $H_{2'}$) ; 5,39 (pseudo t, 1H, $H_{3'}$) ; 4,52 (m, 1H, $H_{4'}$) ; 4,33-4,16 (m, 2H, $H_{5'}$ et $H_{5''}$) ; 2,12-2,05-2 (3s, 9H, 3 $CH_3$ des groupements acétyles).

**Référence** : [1] D.H. Shannahoff et R.A. Sanchez. *J. Org. Chem.*, **38**, 593 (1973).

**2) Synthèse de la (triazole-1,2,4 yl-1)-4 (tri-O-acétyl-2′,3′,5′ α-D-ribofurannosyl)-1 pyrimidinone-2.**

Au composé précédent (24,3 g, 57,5 mmoles) dissous dans de la pyridine anhydre (86 ml) sont ajoutés sous atmosphère d'argon du triazole 1,2,4 (11,9 g, 172,5 mmoles) et du chloro-2 phényl dichlorophosphate (14,2 ml, 86,3 mmoles). La solution est agitée à température ambiante, à l'abri de la lumière pendant 36 heures. Le mélange réactionnel est en suite évaporé sous pression réduite pour donner un résidu marron qui est redissous dans du dichlorométhane (500 ml). Cette solution organique est lavée avec de l'eau (3 × 250 ml) puis avec une solution aqueuse saturée de bicarbonate de sodium (2 × 250 ml) et enfin avec de l'eau (300 ml). Après ces lavages, la solution organique est traitée au noir animal (dichlorométhane à chaud), puis est filtrée. Le filtrat est évaporé sous pression réduite, coévaporé trois fois avec du toluène. On obtient une mousse de couleur orange (20,62 g, 48,81 mmoles). Rendement 85%.

**3) Synthèse de l'α-D-ribofurannosyl-1 cytosine.**

Le composé précédent (20,62 g, 48,81 mmoles) est dissous dans un mélange de dioxanne et d'ammoniaque 32% (3/1 v/v) (580 ml). Le mélange réactionnel est agité à température ambiante pendant trois heures puis la solution est évaporée sous pression réduite. Le résidu obtenu de couleur orangée est redissous dans du méthanol ammoniacal (1,5 l) et la solution est agitée à température ambiante pendant 24 heures. Après évaporation des solvants et coévaporation avec de l'éthanol le produit désiré cristallise. On obtient des cristaux blancs (7 g, 28,7 mmoles). Rendement 60%.

PF : 215°C (éthanol).

U.V. (eau) : $\lambda_{max}$ = 272 nm et $\lambda_{min}$ = 249 nm.

RMN $^1$H (DMSO-$d_6$) δ 7,54 (d, 1H, $H_6$, $J_{5-6}$ = 7,5 Hz) ; 6,02 (d, 1H, $H_{1'}$, $J_{1'-2'}$, 3,84 Hz) ; 5,68 (d, 1H, $H_5$) ; 5,26 (d, 1H, $OH_{2'}$ ou $OH_{3'}$) ; 4,97 (d, 1H, $OH_{2'}$ ou $OH_{3'}$) ; 4,75(m, 1H, $OH_{5'}$) ; 4,05 (m, 2H, $H_{2'}$, et $H_{3'}$), 3,96 (m, 1H, $H_{4'}$) ; 3,61-3,43 (m, 2H, $H_{5'}$ et $H_{5''}$).

**Synthèse de la N-benzoyl-4 α-cytidine[1].**

De l'α-Cytidine (7 g, 28,7 mmoles) est mise en suspension dans de la pyridine anhydre (120 ml). On ajoute sous atmosphère d'argon du chlorure de triméthylsilyle (34 ml, 261 mmoles) et le mélange réactionnel est agité à température ambiante pendant 1 heure 30 minutes. Ensuite du chlorure de benzoyle (4,4 ml, 37,7 mmoles) est ajouté et la solution est agitée pendant trois heures. Un mélange d'eau et de dichlorométhane (55 ml/55 ml v/v) est versé sur la solution préalablement refroidie, on maintient l'agitation pendant 1 heure. La phase organique est évaporée sous pression réduite et coévaporée avec du toluène, puis de l'éthanol. La mousse obtenue est mise en solution dans un mélange eau/éthanol pendant une nuit à température ambiante. Les solvants sont évaporés et coévaporés avec du méthanol. Le produit désiré est obtenu sous forme cristalline avec un rendement de 90% (9 g, 25,9 mmoles).

PF : 207°C (méthanol).

U.V. (éthanol) $\lambda_{max}$ 308 et 261, $\lambda_{min}$ 287 et 229 nm. RMN $^1H$ (DMSO-$d_6$) $\delta$ 11,05 (s, 1H, NH) ; 8,04-7,31 (m, 7H, $H_5$, $H_6$ et H aromatiques du groupement benzoyle) ; 6,09 (d, 1H, $H_{1'}$, $J_{1'-2'}$, 3,95 Hz) ; 5,40 (d, 1H, $OH_{2'}$) ; 5,04 (d, 1H, $OH_{3'}$) ; 4,79 (m, 1H, $OH_{5'}$) ;4,20 (m, 1H, $H_{2'}$) ; 4,09 (m, 2H, $H_{3'}$ et $H_{4'}$) ; 3,69-3,44 (m, 2H, $H_{5'}$ et $H_{5''}$). Spectre de masse (FAB < O ; matrice thioglycérol) : $(M-H)^- = 346$.
**Référence** : [1] W.L. Sung et S.A. Narang, *Can. J. Chem.*, **60**, 111 (1982).

b)synthèse de cytidine alpha entièrement protégée pour l'élongation de l'oligoribonucléotide alpha

b.1. Utilisation du groupement protecteur Ctmp pour le 2'OH du sucre

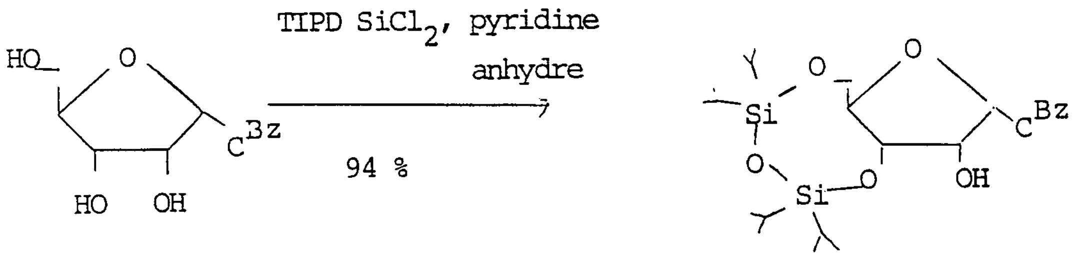


Selon un procédé identique à celui décrit plus haut pour l'obtention de O-(tétraisopropyl-1,1,3,3 disoloxane diyl-1,3)-3',5' $\alpha$-uridine, la O-(tétraisopropyl-1,1,3,3 disoloxane diyl-1,3)-3',5' N-benzoyl-4 $\alpha$-cytidine a été obtenue sous forme d'une poudre incolore après lyophilisation avec un rendement de 94%.
RMN $^1H$ (DMSO-$d_6$) $\delta$ 11,15 (s, 1H, NH) ; 7,34-8,02 (m, 7H, $H_5$, $H_6$ et H aromatiques) ; 6,08 (d, 1H, $H_{1'}$, $J_{1'-2'}$, 3,36 Hz) ; 5,51 (d, 1H, $OH_{2'}$) ; 4,38 (m, 1H, $H_{3'}$) ; 4,28 (m, 1H, $H_{2'}$) ; 4,18 (m, 1H, $H_{4'}$) ; 3,88-4,07 (m, 2H, $H_{5'}$ et $H_{5''}$) ; 0,9-1,14 (m, 28H, CH et $CH_3$ du groupement TIPS).

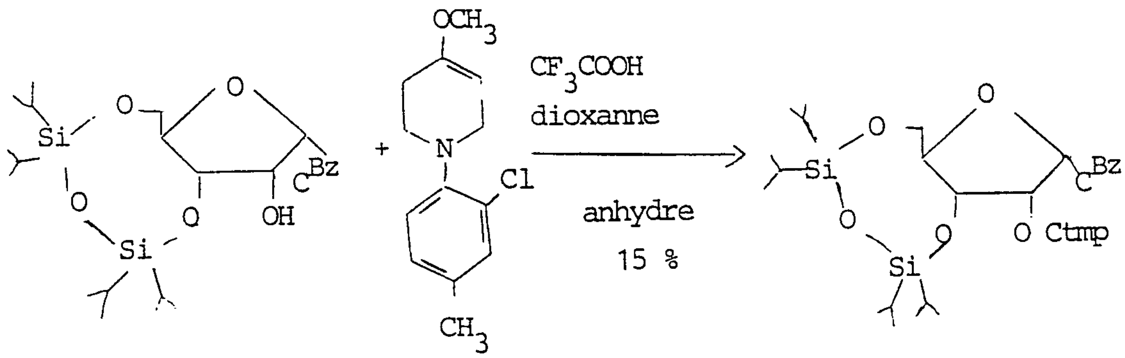


Selon un procédé identique à celui décrit plus haut pour l'obtention de l'analogue de l'$\alpha$-uridine, la O-(tétraisopropyl-1,1,3,3 disiloxane diyl-1,3)-3',5' O-Ctmp-2' N-benzoyl-4 $\alpha$-cytidine a été obtenue sous forme d'une poudre jaune pâle avec un rendement de 15%.
RMN $^1H$ (DMSO-$d_6$) $\delta$ 11,21 (s, 1H, NH), 6,91-8,08 (m, 10H, $H_5$, $H_6$ et H aromatiques) ; 6,29 (d, $H_{1'}$, $J_{1'-2'}$ 3,4 Hz) ; 4,59 (m, 1H, $H_{2'}$) ; 4,48 (m, 1H, $H_{3'}$) ; 4,24 (m, 1H, $H_{4'}$) ; 3,87-4,07 (m, 2H, $H_{5'}$ et $H_{5''}$) ; 3,10 (s, 3H, $OCH_3$) ; 2,93 et 2,63 (2m, 4H, $2CH_2$ du groupement (Ctmp) ; 2,15 (s, 3H, $CH_3$ du groupement Ctmp) ; 1,70-1,94 (m, 4H, $2CH_2$ du groupement Ctmp) ; 0,94-1,08 (m, 28H, CH et $CH_3$ du groupement TIPS).

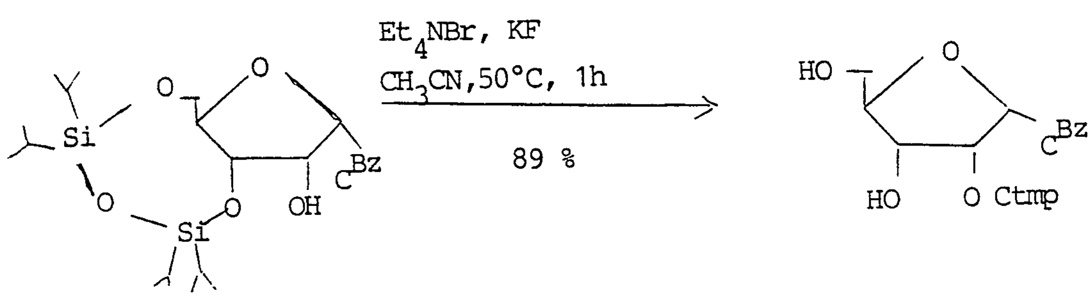


Selon un procédé analogue à celui décrit plus haut pour l'obtention de l'analogue de l'$\alpha$-uridine, la O-Ctmp-2' N-benzoyl-4 $\alpha$-cytidine a été obtenue avec un rendement de 89%.
RMN $^1H$ (DMSO-$d_6$) $\delta$ 11,15 (s, 1H, NH) ; 6,90-8,45 (m, 10H, $H_5$, $H_6$ et H aromatiques ) ; 6,43 (d, 1H, $H'_{1'}$, $J_{1'-2'}$ 6,8

Hz) ; 5,46 (d, 1H, $OH_{3'}$) ; 4,97 (m, 1H, $OH_{5'}$) ; 4,70 (m, 1H, $H_{2'}$) ; 4,18 (m, 2H, $H_{3'}$ et $H_{4'}$) ; 3,49 (m, 2H, $H_{5'}$ et $H_{5''}$) ; 3,19 (s, 3H, $OCH_3$) ; 2,67-2,83 (m, 4H, $2CH_2$ du groupement Ctmp) ; 2,15 (s, 3H, $CH_3$ du groupement Ctmp) ; 1,66-1,85 (m, 4H, $2CH_2$ du groupement Ctmp).

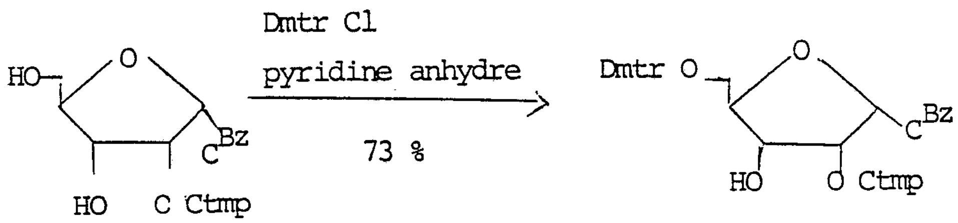


Selon un procédé identique à celui décrit plus haut pour l'obtention de l'analogue de l'α-uridine, la O-diméthoxytrityl-5′ O-Ctmp-2′ N-benzoyl-4 α-cytidine a été obtenu sous forme d'une poudre incolore après précipitation dans de l'éther de pétrole avec un rendement de 73%.
RMN $^1H$ (DMSO-$d_6$) δ 11,15 (s, 1H, NH) ; 6,8-8,5 (m, 23H, $H_5$, $H_6$ et H aromatiques) ; 6,57 (d, 1H, $H_{1'}$, $J_{1'-2'}$ 6,92 Hz) ; 5,54 (d, 1H, $OH_{3'}$) ; 4,91 (pseudo t, 1H, $H_{2'}$) ; 4,32 (m, 1H, $H_{4'}$) ; 4,09 (m, 1H, $H_{3'}$) ; 3,74 (s, 6H, $2OCH_3$ du groupement Dmtr) ; 3,21 (s, 3H, 2 $OCH_3$ du groupement Ctmp) ; 3,04 (m, 2H, $H_{5'}$ et $H_{5''}$) ; 2,60-2,90 (m, 4H, $2CH_2$ du groupement Ctmp) ; 2,15 (s, 3H, $CH_3$ du groupement Ctmp) ; 1,60-1,90 (m, 4H, $2CH_2$ du groupement Ctmp).

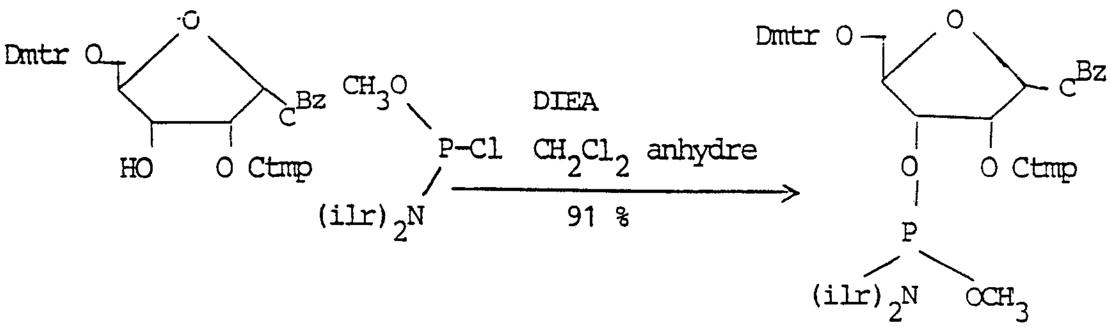


Selon un procédé identique à celui décrit plus haut pour l'obtention de l'analogue de l'α-uridine, le dérivé O-diméthoxytrityl-5′ O-Ctmp-2′ N-benzoyl-4 α-cytidine phosphoramidite-3′ a été obtenu sous forme d'une poudre incolore avec un rendement de 91%.
RMN $^{31}P$ ($CD_3CN$) δ 154,06 et 151,15.

b.2. Utilisation du groupement protecteur TBDMS

**Synthèse de la O-dimethoxytrityl-5′ N-benzoyl-4 α-cytidine.**

A une solution de N-benzoyl-4 α-cytidine précédemment obtenue (9 g, 25,9 mmoles) dans la pyridine anhydre (50 ml) on ajoute du chlorure de diméthoxytrityle (10,1 g, 29,8 mmoles). Le mélange est agité pendant 2 heures à température ambiante puis on ajoute du méthanol (4 ml). Après 5 minutes d'agitation, le mélange réactionnel est versé sur une solution aqueuse saturée de bicarbonate de sodium (350 ml) et les produits sont extraits avec du dichlorométhane (3 × 200 ml). Les phases organiques rassemblées sont séchées sur sulfate de sodium et filtrées. Le filtrat est évaporé sous pression réduite et coévaporé avec du toluène. Le résidu est chromatographié sur une colonne de gel de silice. L'élution est effectuée avec un mélange de méthanol (0 à 10%) dans le dichlorométhane. Les fractions contenant le produit désiré sont regroupées et évaporées à sec. Le résidu est précipité dans l'éther de pétrole. On obtient, après séchage, une poudre blanche (14,5 g, 22,3 mmoles). Rendement 86%.
RMN $^1H$ ($CDCl_3$) δ 8,99 (s large, 1H, NH) ; 8,07-6,75 (m, 20H, $H_5$, $H_6$ et H aromatiques des groupements diméthoxytrityle et benzoyle) ; 6,27 (d, 1H, $H_{1'}$) ; 4,86 (m, 1H, $H_{2'}$) ; 4,38 (m, 1H, $H_{4'}$) ; 4,29 (m, 1H, $H_{3'}$) ; 3,78 (s, 6H, 2 $OCH_3$ du groupement Dmtr) ; 3,47-3,21 (m, 2H, $H_{5'}$ et $H_{5''}$).
Spectre de masse (FAB < 0 ; matrice thioglycérol) : $(M-H)^-$= 648.

**Synthèse de la O-diméthoxytrityl-5′ O-tertiobutyldiméthylsilyl-2′ N-benzoyl-4 α-cytidine.**

A une solution du composé précédent (2,2 g, 3,4 mmoles), dans la pyridine anhydre (33 ml) on ajoute successivement du chlorure de tertiobutyldiméthylsilyle (TBDMS-Cl, 663,1 mg, 4,4 mmoles) et de l'imidazole (601,9 mg, 8,84 mmoles). Le mélange réactionnel est agité à température ambiante pendant 23 heures puis est versé dans une ampoule à décanter contenant une solution aqueuse saturée de bicarbonate de sodium (40 ml). Les produits sont extraits avec du dichlorométhane (2 × 80 ml). Les phases organiques sont

rassemblées et évaporées à sec sous pression réduite et coévaporées avec du toluène. Le résidu est chromatographié sur colonne de gel de silice en utilisant comme éluant un mélange de méthanol (0 à 5%) et de dichlorométhane. Les fractions contenant le produit désiré sont rassemblées et évaporées sous pression réduite. Le résidu est redissous dans du dioxanne puis lyophilisé. On obtient 1,2 g d'une poudre blanche (1,57 mmoles). Rendement 46%.
RMN $^1H$ ($CDCl_3$) δ 8,67 (s, large, 1H, NH) ; 8,01-6,83 (m, 20H, $H_{5'}H_6$, H aromatiques des groupements diméthoxytrityle et benzoyle) ; 6,58 (d, 1H, $H_{1'}$) ; 4,87 (pseudo t, 1H, $H_{2'}$) ; 4,38 (m, 1H, $H_{4'}$) ; 4,19 (m, 1H, $H_{3'}$) ; 3,79 (s, 6H, 2 $OCH_3$ du groupement Dmtr) ; 3,51-3,13 (m, 2H, $H_{5'}$ et $H_{5''}$) ; 2,48 (d, 1H, $OH_{3'}$) ; 0,82 (s, 9H $(CH_3)_3C$ du groupement TBDMS) ; 0,15-0 (2s, 6H, Si $(CH_3)_2$ du groupement TBDMS).

**Synthèse de la O-diméthoxytrityl-5′ O-tertiobutyldiméthylsilyl-2′ O-diisopropylaminométhoxyphosphinyl-3′ N-benzoyl-4 α-cytidine.** (Mélange diastéréoisomérique).

A une solution du composé précédemment obtenu (244 mg, 0,32 mmole) dans du dichlorométhane anhydre (1,2 ml) on ajoute sous atmosphère d'argon de la N,N,N-diisopropyléthylamine (223 µl, 1,28 mmoles) et de la chloro N,N-diisopropylaminométhoxyphosphine (156 µl, 0,8 mmole). Le mélange est agité pendant 20 minutes à température ambiante, sous atmosphère d'argon, puis est versé dans une ampoule à décanter contenant de l'acétate d'éthyle (11 ml). La solution résultante est lavée avec de la saumure (3 × 7 ml). La phase organique est séchée sur sulfate de sodium, filtrée et évaporée à sec sous pression réduite. Le résidu est chromatographié sur colonne de gel de silice et l'élution est effectuée avec des mélanges de cyclohexane, de dichlorométhane et de triéthylamine (90/9/1 à 30/69/1 v/v/v). Les fractions contenant le produit désiré sont rassemblées, évaporées à sec et le résidu redissous dans du dioxanne passé sur alumine est soumis à lyophilisation. On obtient une poudre blanche avec un rendement de 81% (240 mg, 0,259 mmole).
RMN $^{31}P$ ($CD_3CN$) δ 152,40 et 151,26.
RMN $^1H$ ($CDCl_3$) δ 8,60 (massif large, 1H, NH) ; 8,14 (pseudo-t, 1H, $H_6$) ; 7,92-6,79 (m, 18H, H aromatiques des groupements benzoyle et diméthoxytrityle) ; 6,49 et 6,46 (2d, 1H, $H_{1'}$, $J_{1'-2'}$ 4,6 Hz) ; 4,71 (m, 1H, $H_{2'}$) ; 4,44 et 4,38 (2m, 1H, $H_{4'}$) ; 4,33-4,24 (m, 1H, $H_{3'}$) ; 3,77 (s, 6H, 2 $OCH_3$ du groupement Dmtr) ; 3,62-3,47 (m, 2H,2 CH du groupement isopropyle) ; 3,32 et 3,19 (2d, 3H, P-$OCH_{3'}$ $J_{P-H}$ 13,3 Hz) ; 3,43 et 3,11 (m, 2H, $H_{5'}$ et $H_{5''}$) ; 1,18-0,93 (m, 12H, 2$CH_3$ du groupement isopropyle) ; 0,80 et 0,79 (2s, 9H, $(CH_3)_3C$ du groupement TBDMS) ; 0,09 et -0,13 (Ad, 6H, $Si(CH_3)_2$ du groupement TBDMS).
Spectre de masse (FAB < 0 ; matrice polyéthylèneglycol) : $(M-H)^- = 923$.

**EXEMPLE 4 Préparation du synthon phosphoroamidite entièrement protégé de N-benzoyl-6-alpha-adénosine**

**a) Synthèse de la N-benzoyl-6 α-adénosine.**

La N-benzoyl-6 α-adénosine a été obtenue à partir de la N-benzoyl-6 adénine (5 mmoles, 1,2 g) et du tri-O-benzoyl-2,3,5 O-acétyl-1 D-ribofurannose (2,73 g, 5,4 mmoles) selon le procédé de fusion décrit par L. Pichat[1]. La fusion s'effectue en présence d'acide para-toluène sulfonique (100 mg), sous vide (8 mm de mercure), à 180°C pendant 20 minutes. Le résidu obtenu est redissous dans de l'éthanol puis traité au noir animal. La solution décolorée est évaporée et coévaporée au toluène sous pression réduite. Les produits de réaction sont purifiés par chromatographie sur gel de silice. L'élution est effectuée avec des proportions croissantes d'acétone (de 0 à 5%) dans du dichloromethane. Les fractions contenant les deux anomères α et β sont rassemblées et évaporées sous pression réduite. Le résidu est redissous dans une solution de pyridine et d'éthanol (45 ml/27,5 ml v/v) et placé dans un bain de glace. On ajoute de la soude 2N en solution dans un mélange d'eau et d'éthanol (35 ml/35 ml v/v). Le mélange réactionnel est neutralisé par de la DOWEX 50W sous forme pyridinium puis filtré. Le filtrat est évaporé et coévaporé 2 fois avec de l'éthanol. Une première cristallisation dans l'éthanol permet d'éliminer un maximum d'anomère beta. La solution mère s'étant enrichie en anomère alpha, le produit désiré cristallise dans l'éthanol.
PF : 200-201°C (éthanol).
RMN $^1H$ (DMSO-$d_6$) δ 11,15 (s large, 1H, NH) ; 8,73 et 8,64 (2s, 2H, $H_2$ et $H_3$) ; 8,06-7,53 (m, 5H, H aromatiques du groupement benzoyle) ; 6,46 (d, 1H, $H_{1'}$) ; 5,59 (d, 1H, $OH_{2'}$) ; 5,44 (d, 1H, $OH_{3'}$) ; 4,91 (pseudo t, 1H, $OH_{5'}$) ; 4,45 (m, 1H, $H_{2'}$) ; 4,20-4,13 (m, 2H, $H_{3'}$ et $H_{4'}$) ; 3,63-3,39 (m, 2H, $H_{5'}$ et $H_{5''}$).
Spectre de masse (FAB > O ; matrice glycérol): $(M + H)^+ = 372$.
**Référence** : [1] L. Pichat, P. Dufay et Y. Lamorre, *C.R. Acad. Sc. Paris,* 259, 2453 (1964).

**b) Synthèse de la O-diméthoxytrityl-5′ N-benzoyl-6 α-adénosine.**

A une solution de N-benzoyl-6 α-adénosine (960 mg, 2,585 mmoles) dans la pyridine anhydre (13 ml), on ajoute du chlorure de diméthoxytrityle (1,14 g, 3,335 mmoles). Le mélange est agité pendant 3 heures à température ambiante puis on ajoute du méthanol (8 ml). Après 15 minutes d'agitation, le mélange réactionnel est versé sur une solution aqueuse saturée de bicarbonate de sodium (100 ml) et les produits sont extraits avec du dichlorométhane (2 × 100 ml). Les phases organiques rassemblées sont séchées sur sulfate de sodium et filtrées. Le filtrat est évaporé sous pression réduite et coévaporé avec du toluène (3 fois). Le résidu est chromatographié sur une colonne de gel de silice. L'élution est effectuée avec un mélange de méthanol (0 à 3%) dans le dichlorométhane. Les fractions contenant le produit désiré sont regroupées et évaporées à sec. Le résidu est précipité dans de l'éther de pétrole. On obtient, après séchage, une poudre blanche (1,322 g,

1,96 mmoles). Rendement 76%.
RMN $^1H$ ($CDCl_3$) δ 9,25 (s large, 1H, NH) ; 8,53 et 8,21 (2s, 2H, $H_2$ et $H_8$) ; 7,99-6,80 (m, 18H, H aromatiques des groupements diméthoxytrityle et benzoyle) ; 6,43 (d, 1H, $H_{1'}$, $J_{1'-2'}$ 5,99 Hz) ; 5,52 (s large, 1H, $OH_{2'}$) ; 5,27 (s large, 1H, $OH_{3'}$) ; 4,91 (m, 1H, $H_{2'}$) ; 4,53 (m, 1H, $H_{4'}$) ;4,37 (m, 1H, $H_{3'}$) ; 3,76 (s, 6H, 2 $OCH_3$ du groupement Dmtr) ; 3,45-3,16 (m, 2H, $H_{5'}$ et $H_{5''}$).
Spectre de masse (FAB > 0 ; matrice glycérol) : $(M + H)^+ = 674$.

**c) Synthèse de la O-diméthoxytrityl-5′ O-tertiobutyldiméthylsilyl-2′ N-benzoyl-6 α-adénosine.**

A une solution du composé précédent (1,815 g, 2,69 mmoles) précédemment décrit, dans la pyridine anhydre (27 ml) on ajoute successivement du chlorure de tertiobutyldiméthylsilyle (TBDMS-Cl, 576 mg, 3,82 mmoles) et de l'imidazole (478 mg, 7,02 mmoles). Le mélange réactionnel est agité à tempé rature ambiante pendant trois jours. Puis, il est versé dans une ampoule à décanter sur une solution aqueuse saturée de bicarbonate de sodium (100 ml). Les produits sont extraits avec du dichlorométhane (3 × 50 ml). Les phases organiques sont rassemblées, lavées avec de l'eau (200 ml), séchées sur sulfate de sodium et filtrées. Le filtrat est évaporé sous pression réduite puis coévaporé avec du toluène (3 fois). Le résidu est chromatographié sur gel de silice en utilisant comme éluant un mélange de méthanol (0 à 10%) et de dichlorométhane. Les fractions contenant le produit désiré (TBDMS-2′) sont regroupées et évaporées à sec. On obtient une mousse.
Les fractions contenant l'autre isomère (TBDMS-3′) sont regroupées et évaporées à sec. Le résidu (TBDMS-3′, 950 mg) est dissous dans une solution de triéthylamine (0,030 ml) dans du méthanol (12 ml). Le mélange est agité à température ambiante pendant 24 heures puis les solvants sont évaporés sous pression réduite. Le résidu qui est un mélange des deux isomères TBDMS-3′ et TBDMS-2′ est chromatographié sur une colonne de gel de silice. Après élution par un mélange de méthanol (0 à 10%) et de dichlorométhane, les fractions contenant l'isomère substitué en 2′ sont rassemblées et évaporées à sec. Les fractions contenant l'isomère TBDMS-3′ sont soumises à nouveau à un traitement par une solution de triéthylamine dans le méthanol, puis à une purification par chromatographie sur gel de silice comme précédemment. Ainsi, on recueille en trois lots de l'isomère TBDMS-2′ qui, mis en solution dans un dioxanne, est soumis à lyophilisation.
On obtient une poudre blanche (1,17 g, 1,48 mmoles). Rendement 55%.
RMN $^1H$ ($CDCl_3$) δ 9,34 (s, 1H, NH) ; 8,89 et 8,83 (2s, 2H, $H_2$ et $H_8$) ; 8,38-6,84 (m, 18H, H aromatiques des groupements diméthoxytrityle et benzoyle) ; 6,67 (d, 1H, $H_{1'}$, $J_{1'-2'}$ 6,54 Hz) ; 5,07 (t, 1H, $H_{2'}$) ; 4,53 (m, 1H, $H_{4'}$) ; 4,17 (m, 1H, $H_{3'}$) ; 3,80 (s, 6H, 2$OCH_3$ du groupement Dmtr) ; 3,67 (d, 1H, $OH_{3'}$) ; 3,58-3,11 (m, 2H, $H_{5'}$ et $H_{5''}$) ; 0,60 (s, 9H, $(CH_3)_3C$ du groupement TBDMS) ; 0,07 et -0,23 (2s, 6H, $Si(CH_3)_2$ du groupement TBDMS).
Spectre de masse (FAB > 0 ; matrice glycérol) : $(M + H)^+ = 788$.

**d) Synthèse de la O-diméthoxytrityl-5′ O-diisopropylaminométhoxyphosphinyl-3′ O-tertiobutyldiméthylsilyl-2′ N-benzoyl-6 α-adénosine.** (Mélange diastéréoisomérique).

A une solution du composé précédemment obtenu (996,2 mg, 1,264 mmoles) dans du dichlorométhane anhydre (4,8 ml), on ajoute sous atmosphère d'argon de la N,N,N-diisopropyléthylamine (879 µl, 5,05 mmoles) et de la chloro N,N-diisopropylaminométhoxyphosphine (616 µl, 3,16 mmoles). Le mélange réactionnel est agité pendant 30 minutes à température ambiante, sous atmosphère d'argon, puis est versé dans de l'acétate d'éthyle (44 ml). La solution résultante est lavée avec de la saumure (3 × 10 ml). La phase organique est séchée sur sulfate de sodium, filtrée et évaporée sous pression réduite. Le résidu est chromatographié sur colonne de gel de silice et l'élution est effectuée avec des mélanges de cyclohexane, de dichlorométhane et de triéthylamine (99/0/1 à 40/59/1 v/v/v). Les fractions contenant le produit désiré sont rassemblées, évaporées à sec, coévaporées avec du benzène. Le résidu est soumis à lyophilisation dans le benzène. On obtient une poudre blanche (1,049 g, 1,1 mmoles) avec un rendement de 88%.
RMN $^{31}P$ ($CD_3CN$) δ 152,43 et 151,66.
RMN $^1H$ ($CDCl_3$) δ 9,16 et 9,11 (2s, 1H, NH) ; 8,87-6,81 (m, 20H, H aromatiques des groupements diméthoxytrityle et benzoyle, $H_2$ et $H_8$ de la base adénine) ; 6,68 (pseudo-t, 1H, $H_{1'}$, $J_{1'-2'}$ 7 Hz) ; 5,08-4,99 (m, 1H, $H_{2'}$) ; 4,43-4,26 (m, 2H, $H_{4'}$ et $H_{3'}$) ; 3,78 (s, 6H, 2 $OCH_3$ du Dmtr) ; 3,63-3,04 (m, 4H, $H_{5'}$ et $H_{5''}$, 2 CH du groupement isopropyle) ; 3,38 et 3,17 (2d, 3H, P-$OCH_3$, $J_{P-H}$ 13,45 Hz) ; 1,28-0,97 (m, 12H, 2$CH_3$ du groupement isopropyle) ; 0,66 (s, 9H, $(CH_3)_3C$ du groupement TBDMS) ; 0,04 et -0,22 (2d, 6H, $Si(CH_3)_2$ du groupement TBDMS).
Spectre de masse (FAB > 0 ; matrice alcool nitro-benzylique) : $(M + H)^+ = 949$.

**EXEMPLE 5 Synthèse du synthon phosphoroamidite protégé de la N-acétyl-2 α-guanosine**

**1) Synthèse de la O-acétyl-5′ O-isopropylidène-2′,3′ N-acétyl-2 O-diphénylcarbamoyl-6 α-guanosine.**

A une suspension de N-acétyl-2 O-diphénylcarbamoyl-6 guanosine[1] (7,35 g, 18,92 mmoles) dans du dichloroéthane anhydre (150 ml) on ajoute du N,O-Bis(triméthylsilyl)acétamide (8,85 ml, 36,19 mmoles). Le mélange est chauffé à 80°C pendant 15 minutes. Le solvant est distillé sous pression réduite (8 mm de Hg). Sur le résidu huileux obtenu, on ajoute une solution de di-O-acétyl-1′,5′ O-isopropylidène-2′,3′ D-ribofuranose[2] (6,21 g, 22,66 mmoles) dans du toluène anhydre (150 ml) et du triméthylsilyl trifluorométhanesulfonate (6 ml, 31,04 mmoles). Le mélange réactionnel est agité et chauffé à 80°C pendant 1 heure. Puis, il est dilué dans de l'acétate d'éthyle (200 ml) et versé dans de l'eau (200 ml). La phase organique est lavée avec de l'eau (1 fois) et avec une solution aqueuse saturée de bicarbonate de sodium (200 ml). La phase organique est

séchée sur sulfate de sodium et filtrée. Le filtrat est évaporé sous pression réduite. Le résidu est chromatographié sur colonne de gel de silice et l'élution est effectuée avec un mélange acétone (0 à 50%) et de dichlorométhane. Les fractions contenant le produit désiré sont regroupées et évaporées à sec. On obtient une mousse blanche (1,4 g, 2,32 mmoles). Rendement 12,3%.
RMN $^1$H ($CDCl_3$) δ 8,20 (s, 1H, $H_8$) ; 7,95 (s, 1H, NH) ; 7,42-7,21 (m, 10H, H aromatiques du groupement diphénylcarbamoyle) ; 6,42 (d, 1H, $H_{1'}$, $J_{1'-2'}$ 3,8 Hz) ; 4,89-4,83 (m, 2H, $H_{2'}$ et $H_{3'}$) ; 4,55 (m, 1H, $H_{4'}$) ; 4,31-4,19 (m, 2H, $H_{5'}$ et $H_{5''}$) ; 2,52 (s, 3H, $CH_3$ du groupement acétyle sur le ribose) ; 2,14 (s, 3H, $CH_3$ du groupement acétyle sur la base guanine) ; 1,48 et 1,49 (2s, 6H, $2CH_3$ du groupement isopropylidène sur le sucre).
Référence : [1] R. Zou, M.J. Robins, *Can. J. Chem.* 65, 1436 (1987).
[2] P.A. Levene, E.T. Stiller, *J. Biol. Chem.* 102, 187 (1933).

**2) Synthèse de la O-isopropylidène-2′,3′ N-acétyl-2 α-guanosine.**

A une solution du composé précédemment décrit (1,45 g, 2,4 mmoles) dans du tétrahydrofuranne anhydre (12 ml), on ajoute une solution 1M de fluorure de tétrabutylammonium dans le tétrahydrofuranne (12 ml, 12 mmoles). Le mélange réactionnel est agité à température ambiante pendant 20 heures. Le solvant est évaporé sous pression réduite. Le résidu obtenu est dissous dans de l'acétate d'éthyle (50 ml) puis le mélange est versé dans de l'eau (50 ml). La phase organique est lavée avec de l'eau (2 × 50 ml). Le produit désiré est dans la phase aqueuse que l'on évapore sous pression réduite. Le résidu est chromatographié sur gel de silice greffée RP2 (Merck n° 7719), l'élution est effectuée avec un mélange d'eau et de méthanol (100/0 à 50/50 v/v). Les fractions contenant le produit désiré sont rassemblées, évaporées à sec. Après lyophilisation on obtient une poudre blanche (581 mg, 1,6 mmoles). Rendement 66%.
RMN $^1$H (DMSO-$d_6$) δ 11,95 (s large, 2H, 2NH); 7,89 (s, 1H, $H_8$) ; 6,32 (d, 1H, $H_{1'}$) ; 5,25 (s large, 1H, $OH_{5'}$) ; 4,90 (m, 1H, $H_{3'}$) ; 4,80 (m, 1H, $H_{2'}$) ; 4,35 (M, 1H, $H_{4'}$) ; 3,61 (m, 2H, $H_{5'}$ et $H_{5''}$) ; 2,14 (s, 3H, $CH_3$ du groupement acétyle sur la base guanine) ; 1,31 et 1,23 (2s, 6H, $2CH_3$ du groupement isopropylidène sur le sucre).
Spectre de masse (FAB > O ; matrice thioglycérol) : $(M + H)^+ = 366$.

**3) Synthèse de la N-acétyl-2 α-guanosine.**

Le produit précédemment décrit (5,81 mg, 1,6 mmoles) est soumis à un traitement acide avec un mélange d'acide formique et d'eau (50/50 v/v) (8 ml). Le mélange réactionnel est agité à température ambiante pendant quatre jours. Puis, les solvants sont évaporés sous pression réduite, le résidu obtenu est coévaporé plusieurs fois avec de l'eau (10 fois). Le produit désiré cristallise dans l'eau. On obtient des cristaux blancs (330 mg, 1,016 mmoles). Rendement 73%.
RMN $^1$H (DMSO-$d_6$) δ 12,00 et 11,70 (2s large, 2H, 2NH) ; 8,14 (s, 1H, $H_8$) ; 6,09 (d, 1H, $H_{1'}$, $J_{1'-2'}$ 5,27 Hz) ; 5,52 (d, 1H, $OH_{2'}$) ; 5,36 (d, 1H, $OH_{3'}$) ; 4,89 (t, 1H, $OH_{5'}$) ; 4,33 (m, 1H, $H_{2'}$) ; 4,12 (m, 1H, $H_{3'}$) ; 4,08 (m, 1H, $H_{4'}$) ; 3,58-3,41 (m, 2H, $H_{5'}$ et $H_{5''}$) ; 2,17 (s, 3H, $CH_3$ du groupement acétyle sur la base guanine).
Spectre de masse (FAB > O ; matrice thioglycérol) : $(M + 2H)^+ = 327$.

**Synthèse de la O-diméthoxytrityl-5′ N-acétyl-2 α-guanosine.**

A une solution de N-acétyl-2 α-guanosine (330 mg, 1,016 mmoles) dans la pyridine anhydre (6,5 ml), on ajoute du chlorure de diméthoxytrityle (489 mg, 1,445 mmoles). Le mélange est agité pendant 3 heures à température ambiante puis on ajoute du méthanol (1 ml). Après 5 minutes d'agitation, le mélange réactionnel est versé sur une solution aqueuse saturée de bicarbonate de sodium (25 ml) et les produits sont extraits avec du dichlorométhane (3 × 25 ml). Les phases organiques rassemblées sont lavées avec de l'eau (50 ml), séchées sur sulfate de sodium et filtrées. Le filtrat est évaporé sous pression réduite et coévaporé avec du toluène (3 fois).
Le produit désiré cristallise dans le dichlorométhane. On obtient des cristaux blancs (532 mg, 0,848 mmole). Rendement 83,5%.
PF : 242°-243° C.
RMN $^1$H (DMSO-$d_6$) δ 12,04 et 11,70 (2s large, 2H, 2NH) ; 8,18 (s, 1H, $H_8$) ; 7,41-6,83 (m, 13H, H aromatiques du groupement diméthoxytrityle) ; 6,18 (d, 1H, $H_{1'}$, $J_{1'-2'}$ 5,18 Hz) ; 5,63 (s large, 1H, $OH_{2'}$) ; 5,42 (s large, 1H, $OH_{3'}$) ; 4,40 (m, 1H, $H_{2'}$) ; 4,27 (m, 1H, $H_{4'}$) ; 4,14 (m, 1H, $H_{3'}$) ; 3,74 (s, 6H, 2 $OCH_3$ du groupement Dmtr) ; 3,32-3,00 (m, 2H, $H_{5'}$ et $H_{5''}$) ; 2,18 (s, 3H, $CH_3$ du groupement acétyle sur la base guanine).
Spectre de masse (FAB < 0 ; matrice thioglycérol) : $(M-H)^- = 626$.

**Synthèse de la O-diméthoxytrityl-5′ O-tertiobutyldiméthylsilyl-2′ N-acétyl-2 α-guanosine [5].**

A une solution du composé (518 mg, 0,826 mmole) précédemment décrit, dans la pyridine anhydre (8,3 ml) on ajoute successivement du chlorure de tertiobutyldiméthylsilyle (TBDMS-Cl, 162 mg, 1,074 mmoles) et de l'imidazole (125 mg, 2,147 mmoles). Le mélange réactionnel est agité à température ambiante pendant quatre jours. Puis, il est versé dans une ampoule à décanter sur une solution aqueuse saturée de bicarbonate de sodium (25 ml). Les produits sont extraits avec du dichlorométhane (3 × 25 ml). Les phases organiques sont rassemblées, lavées avec de l'eau (50 ml), séchées sur sulfate de sodium et filtrées. Le filtrat est évaporé sous pression réduite puis coévaporé avec du toluène (3 fois). Le résidu est chromatographié sur gel de silice en utilisant comme éluant un mélange de méthanol (0 à 20%) et de dichlorométhane. Les fractions contenant le produit désiré (TBDMS-2′) sont regroupées et évaporées à sec. On les soumet à lyophilisation dans le dioxanne (124 mg). Les fractions contenant le mélange des deux isomères (TBDMS-2′ et TBDMS-3′) sont

regroupées, évaporées à sec puis dissoutes dans 2,5 ml d'éthanol. Le mélange est agité à température ambiante pendant 20 heures, puis le solvant est évaporé sous pression réduite. Le résidu est chromatographié sur une colonne de gel de silice. Après élution par un mélange de méthanol (0 à 20%) et de dichlorométhane, les fractions contenant l'isomère TBDMS-2′ sont rassemblées et évaporées à sec. Les fractions riches en isomère TBDMS-3′ sont soumises à nouveau à isomérisation dans l'éthanol, puis à une purification par chromatographie sur gel de silice comme précédemment. Ainsi, on recueille en trois lots de l'isomère TBDMS-2′ qui, mis en solution dans du dioxanne, est soumis à lyophilisation. On obtient une poudre blanche (250 mg, 0,342 mmole). Rendement 42%.
RMN $^1H$ ($CDCl_3$) $\delta$ 11,87 (s large, 1H, NH) ; 8,36 (s large, 1H, NH) ; 8,00 (s, 1H, $H_8$) ; 7,42-6,83 (m, 13H, H aromatiques du groupement Dmtr) ; 6,25 (d, 1H, $H_{1'}$, $J_{1'-2'}$ 6,59 Hz) ; 4,93 (t, 1H, $H_{2'}$) ; 4,42 (m, 1H, $H_{4'}$) ; 4,16 (m, 1H, $H_{3'}$) ; 3,79 (s, 6H, 2 $OCH_3$ du groupement Dmtr) ; 3,52-3,12 (m, 2H, $H_{5'}$ et $H_{5''}$) ; 3,32 (d, 1H, $OH_{3'}$) ; 2,28 (s, 3H, $CH_3$ du groupement acétyle sur la base guanine) ; 0,69 (s, 9H, $(CH_3)_3C$ du groupement TBDMS) ; 0,04 et -0,19 (2s, 6H, 2$CH_3$ du groupement TBDMS).
Spectre de masse (FAB < O ; matrice thioglycérol) : $(M-H)^- = 740$.

**Synthèse de la O-diméthoxytrityl-5′ O-diisopropylaminométhoxyphosphinyl-3′ O-tertiobutyldiméthylsilyl-2′ N-acétyl-2 α-guanosine.** (Mélange diastéréoisomérique).

A une solution de N,N,N-diisopropyléthylamine (190 µl, 1,078 mmoles) et de chloro N,N-diisopropylamino-méthoxyphosphine (130 µl, 0,647 mmole) dans du dichlorométhane anhydre (200 µl), on ajoute goutte à goutte, sous atmosphère d'argon et sous agitation, une solution du composé précédemment décrit **[5]** (200 mg, 0,269 mmole) dans du dichlorométhane anhydre (800 µl). Le mélange réactionnel est agité pendant 1 h 15 minutes à température ambiante puis est versé dans de l'acétate d'éthyle (3 ml). La solution résultante est lavée avec de la saumure (3 × 7 ml). La phase organique est séchée sur sulfate de sodium, filtrée et évaporée sous pression réduite. Le résidu est chromatographié sur colonne de gel de silice et l'élution est effectuée avec des mélanges d'acétonitrile, de dichlorométhane et de triéthylamine (0/99/1 à 50/50/1 v/v/v). Les fractions contenant le produit désiré sont rassemblées, évaporées à sec, coévaporées avec du benzène. Le résidu est soumis à lyophilisation dans le benzène. On obtient une poudre blanche (192 mg, 0,212 mmole) avec un rendement de 79%.
RMN $^{31}P$ ($CD_3CN$) $\delta$ 152,47 et 151,37.
RMN $^1H$ ($CDCl_3$) $\delta$ 11,82 (s large, 1H, NH) ; 8,39 et 8,32 (2s, 1H, $H_8$) ; 8,32 (s large, 1H, NH) ; 7,45-6,83 (m, 13H, H aromatiques du groupement Dmtr) ; 6,12 (pseudo-t, 1H, $H_{1'}$, $J_{1'-2'}$ 6,8 Hz) ; 4,93 (m, 1H, $H_{2'}$) ; 4,34 (m, 2H, $H_{3'}$ et $H_{4'}$) ; 3,80 (s, 6H, 2 $OCH_3$ du groupement Dmtr) ; 3,61-3,51 (m, 2H, 2 CH du groupement isopropyle) ; 3,49-3,10 (m, 2H, $H_{5'}$ et $H_{5''}$) ; 3,40 et 3,18 (2d, 3H, $OCH_3$ sur le phosphore, $J_{P-H} = 13,3$ Hz) ; 2,28 et 2,27 (2s, 3H, $CH_3$ du groupement acétyle sur la base guanine) ; 1,25-0,99 (m, 12H, 4$CH_3$ du groupement isopropyle) ; 0,72 (2s, 9H, $(CH)_3C$ du groupement TBDMS) ; 0,05 et -0,15 (2s, 6H, 2$CH_3$ du groupement TBDMS).
Spectre de masse (FAB < 0 ; matrice polyéthylèneglycol) : $(M-H)^- = 901$.

**EXEMPLE 6 Synthèse d'oligoribonucléotides alpha sur support solide par la méthode au phosphoramidite (échelle = 1 µmole)**

1. Synthèse de alpha-['Up)$_7$U] sur support solide par la méthode au phosphoroamidite

a) Conditions de synthèse
* Synthon phosphoroamidite rU alpha utilisé dans la synthèse de alpha-[(Up)$_7$U]

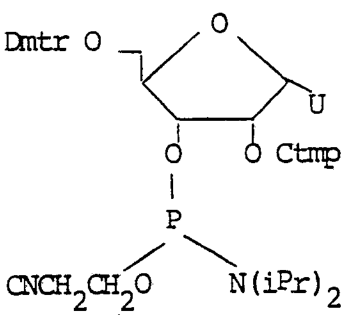

Concentration utilisée = 0.2 M(20e)

* Agent activateur utilisé pour les condensations : le p-nitro phényl tétrazole à la concentration de 0.2 M (40e) (soluble à chaud environ 45°C)

b)Cycle de synthèse et rendements de condensation

Etapes impliquées dans un cycle d'élongation

| N° | Etape | Solvant ou Réactif | Temps(s) |
|---|---|---|---|
| 1 | Lavage et Purge | $CH_3CN$, Argon | 52 |
| 2 | Détritylation | 3% TCA dans $CH_2Cl_2$ | 100 |
| 3 | Lavage et Purge | $CH_3CN$, Argon | 225 |
| 4 | Condensation | 0.2M amidite (20eq) dans $CH_3CN$ + 0.2 M $pNO_2$ phényl tétrazole (40e) dans $CH_3CN$ | 931 |
| 5 | Purage | Argon | 10 |
| 6 | Cappage | 6,5 % DMAP dans THF + $Ac_2O$/lutidine/THF 1 1 8 | 143 |
| 7 | Purage | Argon | 20 |
| 8 | Oxydation | 0.1 M $I_2$ dans THF/Pyr/$H_2O$ (40/10/1) | 55 |
| 9 | Lavage | $CH_3CN$ | 110 |

Les rendements de condensation sont calculés par le dosage spectrophotométrique à $\lambda$ = 498 nm du cation diméthoxytrityl libéré au cours de l'étape de détritylation.
Rendement global : 70 %
Rendement moyen par étape = 95 %

c) déprotection et purification d'alpha-[$(Up)_7U$]
- décrochage du support par $NH_4OH$ 32 % pendant 3 fois 30 min,
- traitement par $NH_4OH$ 32 % pendant 1 heure à 55°C (élimination du groupment Benzoyle présent sur le premier nucléoside fixé au support),
- traitement par HCl O.O1 M (pH = 2) pendant 24 heures à température ambiante (élimination du Ctmp),
- purification par colonne DEAE-Sephadex. On obtient 19 unités d'absorbance à 260 nm,
- l'analyse par HPLC du mélange brut obtenu indique une pureté de 82 %.

2. Synthèse de alpha-[$(Up)_5U$] sur support solide par la méthode au phosphoroamidite

a) Conditions de synthèse
* synthon phosphoroamidite rU alpha utilisé dans la synthèse de alpha-[$(Up)_5U$].

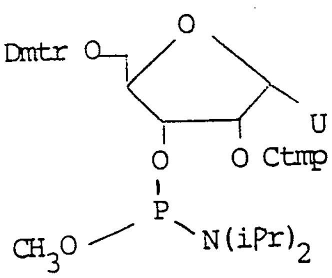

Concentration utilisée : 0.2 M

* agent activateur utilisé pour les condensations : le p-nitrophényl tétrazole à la concentration de O.2 M (soluble à chaud environ 45°C)

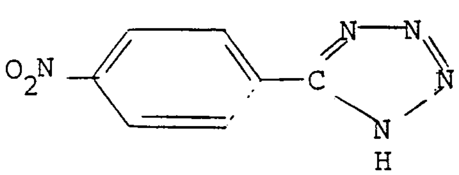

b) Cycle de synthèse et rendements de condensation

Etapes impliquées dans un cycle d'élongation

| N° | Etape | Solvant ou Réactif | Temps(s) |
|---|---|---|---|
| 1 | Lavage et Purge | $CH_3CN$, Argon | 52 |
| 2 | Détritylation | 3% TCA dans $CH_2Cl_2$ | 100 |
| 3 | Lavage et Purge | $CH_3CN$, Argon | 231 |
| 4 | Condensation | 0.2M amidite (20eq) dans $CH_3CN$ + 0.2 M $pNO_2$ phényl tétrazole (40e) dans $CH_3CN$ | 937 |
| 5 | Purge | Argon | 10 |
| 6 | Cappage | 6,5 % DMAP dans THF + $Ac_2O$/lutidine/THF 1 / 1 / 8 | 143 |
| 7 | Purge | Argon | 20 |
| 8 | Oxydation | 0.1 M $I_2$ dans THF/Pyr/$H_2O$ (40/10/1) | 55 |
| 9 | Lavage | $CH_3CN$ | 110 |

Rendement moyen par étape : 97 %Rendement global pour alpha-[$(Up)_5U$] : 85 %

c) déprotecteur et purification d'alpha-[$(Up)_5U$]
- déprotection des phosphates par une solution thiophénol $Et_3N$/dioxanne (1/1/2 v/v/v) pendant 30 min à température ambiante,
- décrochage de l'oligoribonucléotide sur le support par $NH_4$ OH 32 % pendant 3 fois 30 minutes à température ambiante, puis chauffage à 55° C pendant 1 heure,
- déprotection des 2'OH des sucres par une solution HCl 0.01 M pendant 24 heures à température ambiante. Quantité d'oligoribonucléotide alpha-[$(Up)_5U$] obtenue = 42 unités d'absorbance à 260 nm,
- purification par colonne DEAE-Sephadex. Quantité d'oligonucléotide alpha obtenue = 12 unités d'absorbance à 260 nm,
- analyse HPLC de $rU_6$ alpha pour vérification de pureté : 74 %.

2. Synthèse de alpha-[$r(Up)_{11}u$] sur support solide par la méthode au phosphoroamidite

(a) Conditions de synthèse :

. Synthon phosphoroamidite rU alpha utilisé dans la synthèse de alpha-[$r(Up)_{11}U$]

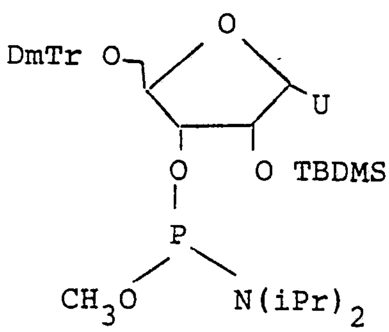

Concentration utilisée :
0.15 M (20 eq.)

b) Cycle de synthèse et rendements de condensation :

*Etapes impliquées dans un cycle d'élongation*

| N° | ETAPE | SOLVANT ou REACTIF | TEMPS(s) |
|---|---|---|---|
| 1 | Lavage et Purge | $CH_3CN$ , Argon | 52 |
| 2 | Détritylation | 3 % TCA dans $CH_2Cl_2$ | 100 |
| 3 | Lavage et Purge | $CH_3CN$, Argon | 225 |
| 4 | Condensation | 0.15M amidite (20eq.) dans $CH_3CN$ + 0.5M tétrazole (50 eq.) dans $CH_3CN$ | 920 |
| 5 | Purge | Argon | 10 |
| 6 | Cappage | 6,5 % NMI* dans THF + $Ac_2O$/lutidine/THF 1 1 8 | 93 |
| 7 | Purge | Argon | 20 |
| 8 | Oxydation | 0.1M $I_2$ dans THF/Pyr/$H_2O$ (40/10/1) | 65 |
| 9 | Lavage | $CH_3CN$ | 100 |

* N-Méthyl-Imidazole

Les rendements de condensation sont calculés par le dosage spectrophotométrique à $\lambda$ = 498 nm du cation diméthoxytrityl libéré au cours de l'étape de détritylation.*Rendement global : 78 %*
*Rendement moyen par étape : 97,8 %*

c) Déprotection et purification d'alpha-[$(Up)_{11}U$] :
- traitement avec une solution de thiophénol-triéthylamine-dioxane (1/1/2, v/v/v) pendant 30 minutes.
- décrochage du support par un mélange d'ammoniaque 32 % et d'éthanol (3/1, v/v) pendant 3 fois 30 minutes et incubation du surnageant pendant 3 heures à 55°C (élimination du groupement benzoyle présent sur le premier nucléoside fixé au support) .
- évaporation sous pression réduite et traitement du résidu par une solution 0.1M de tétrabutylammonium dans le tétrahydrofuranne pendant 18 heures.
- purification par colonne DEAE-Sephadex. On obtient 40 unités d'absorbance à 260 nm. L'analyse par HPLC indique une pureté de 90 %.

**EXEMPLE 7 Propriétés biophysiques**

**I - Mise en évidence de l'hybridation de l'oligoribonucléotide $\alpha$-[$(Up)_{11}$**U] avec du poly A.

Les expériences d'hybridation ont été effectuées à l'aide d'un spectrophotomètre Uvikon 810 (Kontron) interfacé avec un micro-ordinateur IBM PC compatible. Le contrôle de la température a été opéré à l'aide d'un programmateur de température Huber PD415 connecté à un bain d'eau réfrigéré à circulation (Huber Ministat). Les cellules ont un trajet optique de 1 cm et leur compartiment est balayé par un flux d'azote tout au long de l'expérience.

Auparavant, les deux brins d'oligonucléotides étudiés sont mélangés et chauffés à 80°C pendant 30 minutes. Les valeurs digitalisées d'absorbance et de température sont accumulées par l'ordinateur. Dans une expérience typique, l'ordinateur collecte les valeurs chaque 30 secondes alors que la variation de température est de 0,75°C/min. 200 points sont donc collectés pour chaque courbe entre 5°C et 80°C. La formation ou la dénaturation de l'hybride se traduit par une variation rapide de l'absorbance (à 260 nm dans nos expériences). Les Tm correspondent à la température de demi-transition (point d'inflexion).

L'oligonucléotide $\alpha$-[$(Up)_{11}U$] et le poly (A) ont été utilisés à une concentration (exprimée en base) de 240 $\mu$M dans un tampon NaCl (10 mM), cacodylate de sodium (10 mM) ajusté à pH 7,0.

Dans ces conditions, on obtient une température de demi-transition :

$T_m$ = 16°C

Conclusion : les ARN alpha ont un appariemment très stable avec les ARN beta.

Les applications biologiques qui peuvent en découler sont donc nombreuses notamment à titre de sondes d'acides nucléiques ou a titre d'agents thérapeutiques.

**II - Comparaison de la stabilité des oligoribonucléotides $\alpha$-et $\beta$-[$(Up)_5U$] vis-à-vis de nucléases.**

Conditions d'analyses HPLC.

Les analyses ont été effectuées sur colonne Radial-Pak $C_{18}$ (10 $\mu$m) placée dans un module RCM-100 (Waters). Le matériel se compose d'un injecteur U6K (Waters), de deux pompes 6000A (Waters), d'un programmateur de gradient M720 (Waters), d'un détecteur multicannal Pye Unicam PU 4021 et d'une console de contrôle Pye Unicam PU 4850. L'élution est effectuée selon un gradient linéaire (de 0 à 12,5%) d'acétonitrile dans un tampon d'acétate d'ammonium 0,1M (pM 5,9) et à un débit de 3 ml $min^{-1}$. La détection est effectuée à 254 nm.

**1.** Dégradation par la phosphodiesterase de venin de *crotalus durissus terrificus* (3'-exonucléase).

Une solution d'oligonucléotide (20 μℓ, 2 unités d'absorbance à 260 nm) est diluée dans un tampon Tris-HCl 0,1 M (pH 9), $MgCl_2$ 0.01 M (100 μℓ). On ajoute une solution commerciale de phosphodiestérase (2 μℓ, 3 unités/ml). Le mélange est incubé à 37°C. A des temps déterminés, des fractions aliquotes (10 μℓ) sont prélevées, chauffées à 100°C pendant 2 minutes et analysées.

| % de substrat restant / Temps d'incubation | 2mn | 120 mn |
|---|---|---|
| rU6 beta | 6,5 % | 0 |
| rU6 alpha | 94 % | 42 % |

**2.** Dégradation par la phosphodiestérase de rate de veau (5'-exonucléase).

Une solution d'oligonucléotide (20 μℓ, 2 unités d'absorbance à 260 nm) est dilué dans un tampon d'acétate d'ammonium 0,125 M (pH 7,0), d'EDTA 2,5 mM et de Tween 80 0,0625% (80 μℓ). On ajoute une solution commerciale de phosphodiesterase (2 μℓ, concentration finale 0,013 unités/ml) et le mélange est incubé à 37°C. A des temps déterminés, des fractions aliquotes sont prélevées (10 μℓ), chauffées à 100°C pendant 2 minutes et analysées.

| % de substrat restant / Temps d'incubation | 2mn | 60 mn |
|---|---|---|
| rU6 beta | 89 % | 13 % |
| rU6 alpha | 100 % | 100 % |

**3.** Dégradation enzymatique par le nucléase S1 de *Aspergillus Oryzae* (endonucléase spécifique du simple brin).

A une solution d'oligonucléotide (20 μℓ, 2 unités d'absorbance à 260 nm) on ajoute du tampon acétate d'ammonium 0,5 M (pH 4,7), NaCl 3M et acétate de zinc 0.1 M (10 μℓ) et de l'eau (70 μℓ). On ajoute une solution d'ensyme (2 μℓ, 2 unités, obtenue par dilution d'une solution commerciale dans le même tampon). Le mélange est incubé à 37°C. A des temps déterminés, des fractions aliquotes (10 μℓ) sont prélevées, diluées dans du tampon phosphate de potassium 0,1M (pH 6,5, 10 μℓ) et analysées.

| % de substrat restant / temps d'incubation | 1 heure | 5 heures |
|---|---|---|
| rU6 beta | 68 % | 14 % |
| rU6 alpha | 100 % | 100 % |

**4.** Dégradation enzymatique par la RNase A de pancréas de bovin (ribonucléase).

Une solution d'oligonucléotide (20 μℓ, 2 unités d'absorbance à 260 nm) est diluée dans un tampon NaCl 0,3M, Tris 0,01M (pH 7,4) et EDTA 0,005M (120 μℓ). On ajoute une solution d'enzyme (2 μℓ, $4.10^{-2}$ unités), et le mélange est incubé à 37°C. A des temps déterminés, des fractions aliquotes (50 μℓ) sont prélevées et immédiatement analysées.

| % de substrat restant / Temps d'incubation | 5 mn | 35 mn |
|---|---|---|
| rU6 beta | 0,3 % | 0 % |
| rU6 alpha | 100 % | 100 % |

Conclusion : les oligoribonucléotides alpha sont beaucoup plus stables vis-à-vis de la résistance aux nucléases que les oligoribonucléotides beta correspondants.

**Revendications**

1. Procédé de synthèse de composés oligoribonucléotides alpha caractérisé en ce qu'on effectue une synthèse du type au phosphoramidite sur support solide.

2. Procédé selon la revendication 1, caractérisé en ce qu'on assemble des monomères ribonucléosides alpha protégés, substitués par des groupes phosphoramidites en 3′, la fonction hydroxyle en 2′ du ribose dudit monomère étant protégée par le groupe Ctmp ou TBDMS.

3. Procédé selon la revendication 1 ou 2, caractérisé en ce que un composé ribonucléoside alpha protégé est tout d'abord immobilisé sur un support solide par l'intermédiaire d'une de ses fonctions hydroxyle en 2′ ou 3′.

4. Procédé selon la revendication 3, caractérisé en ce que ledit composé ribonucléoside alpha immobilisé a une fonction hydroxyle libre 3′ ou 2′ respectivement, protégée par un groupe protecteur tel que le groupe benzoyle.

5. Procédé selon l'une des revendications 2 à 4, caractérisé en ce que l'assemblage de l'oligoribonucléotide alpha se fait par condensation, en présence d'un agent activateur, desdits monomères entre leur fonction en 3′ et la fonction 5′ du composé ribonucléoside alpha immobilisé pour le premier monomère ou d'un composé intermédiaire polyribonucléotide alpha protégé fixé sur ledit composé ribonucléoside alpha immobilisé pour les monomères suivants.

6. Procédé selon l'une des revendications 2 à 5, caractérisé en ce que le groupe phosphoramidite en 3′ desdits monomères est un groupe N, N dialkylaminophosphoramidite de formule (I)

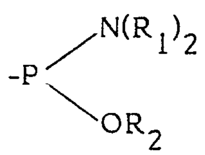

avec $R_1$ qui représente un alkyl en $C_1$ à $C_7$ éventuellement substitué, identique ou différent, tel que $CH_3$, $(CH_3)_2CH$,

$R_2$ qui représente un alkyl en $C_1$ à $C_7$ éventuellement substitué tel que $CH_3$, $CH_2CH_2CN$.

7. Procédé selon la revendication 6, caractérisé en ce que le groupe phosphoramidite en 3′ desdits monomères et le diisopropylaminophosphoramidite de méthyle ($R_1 = (CH_3)_2CH$ et $R_2 = CH_3$) ou le diisopropylaminophosphoramidite de cyano-2 éthyle $R_1 = (CH_3)_2CH$ et $R_2 = CH_2CH_2CN$).

8. Procédé selon l'une des revendications 3 à 7, caractérisé en ce que les groupes hydroxyle desdits monomères et dudit composé ribonucléoside alpha immobilisé sont protégés en 5′ par le groupe Dmtr.

9. Procédé selon l'une des revendications 4 à 8, caractérisé en ce que l'agent activateur pour la condensation desdits monomères est choisi parmi le tétrazole et ses dérivés, tels que le paranitro phényl tétrazole.

10. Procédé selon l'une des revendications 3 à 9, caractérisé en ce que le composé ribonucléoside alpha immobilisé est fixé sur le support solide via un groupe divalent du type succinyle entre un de ses groupements OH en 2′ ou 3′ qui se trouve ainsi estérifié, et un groupement amino du support.

11. Procédé selon l'une des revendications précédentes, caractérisé en ce que le support solide est constitué par une longue chaîne alkylamine fixée sur un polymère, un gel de silice ou des billes de verre poreuses.

12. Composés utiles dans le procédé selon l'une des revendications 1 à 11, caractérisés en ce qu'ils consistent et un monomère alpha ribonucléoside substitué par un groupe phophoramidite en 3' et dont la fonction hydroxyle en 2' est protégée par un groupe protecteur tel que le groupe Ctmp ou TBDMS.

13. Composés utiles dans le procédé selon l'une des revendications 1 à 11, caractérisé en ce qu'il répondent à la formule (II)

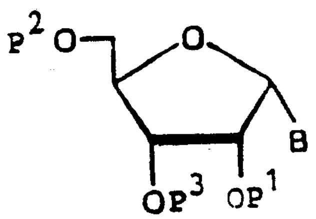

dans laquelle
B est une base rattachée au cycle glycosidique selon une configuration anomérique alpha choisie parmi U, A, C, G, et lesdites bases modifiées et/ou protégées.
$P^1$ un groupe protecteur de la fonction hydroxyle telle que Ctmp, TBDMS.
$P^2$ H, un groupe protecteur de la fonction hydroxyle telle que Dmtr.
$P^3$ un groupe phosphoramidite tel qu'un groupe N,N dialkylaminophosphoramidite de formule (I)

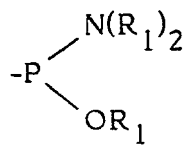

(I)

avec $R_1$ qui représente un alkyl en $C_1$ à $C_7$ éventuellement substitué, identique ou différent, tel que $CH_3$, $(CH_3)_2CH$,
$R_2$ qui représente un alkyl en $C_1$ à $C_7$ éventuellement substitué tel que $CH_3$, $CH_2CH_2CN$.

14. Composés utiles dans le procédé selon l'une des revendications 1 à 11, caractérisés en ce qu'ils consistent en un ribonucléoside alpha immobilisé sur un support solide par l'intermédiaire de sa fonction hydroxyle en 2' ou 3'.

15. Composés utiles dans le procédé selon l'une des revendications 1 à 11, caractérisés en ce qu'ils répondent à la formule générale (III)

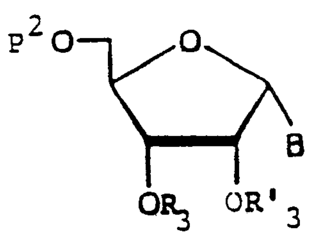

(III)

dans laquelle
B et $P^2$ ont les significations données précédemment.
- L'un de $R_3$ et $R'_3$ représente H ou un groupement protecteur de la fonction hydroxyle tel que le groupement benzoyl,
- l'autre de $R_3$ et $R'_3$ représentant un groupe fonctionnel permettant la fixation à un support solide par l'intermédiaire d'une fonction terminale, ou ledit groupe fonctionnel lié au support solide.

16. Composés selon la revendication 14, caractérisés en ce que $R_3$ ou $R'_3$ représente le groupe de formule

$$-\underset{\underset{O}{\|}}{C}-(CH_2)_p-\underset{\underset{O}{\|}}{C}R_4 \qquad (IV)$$

avec $p = 1$ à 5
avec $R_4$ : qui représente OH, OH activé par un groupe d'activation tel que $-C_6Cl_5$, ou un radical -NH lié à un support solide tel que le radical -NH d'une chaîne alkylamine fixée sur polymère, gel de silice ou billes de verre poreuses.

17. Composés oligoribonucléotides alpha obtenus par le procédé selon l'une des revendications 1 à 11.

18. Composés selon la revendication 17, caractérisés en ce qu'ils ne comportent pas uniquement les bases U et C.

Office européen des brevets

# RAPPORT DE RECHERCHE EUROPEENNE

Numero de la demande

EP 89 40 1681

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | WO-A-8 804 301 (CNRS)<br>* Exemples 3,4 *<br>--- | 1 | C 07 H 19/067<br>C 07 H 19/167<br>C 07 H 21/02 |
| Y | JOURNAL OF THE AMERICAN CHEMICAL SOCIETY, vol. 109, 1987, pages 7845-7854, American Chemical Society, Columbus, Ohio, US; N. USMAN et al.: "Automated chemical synthesis of long oligoribonucleotides using 2'-O-silylated ribonucleoside 3'-O-phosphoramidites on a controlled-pore glass support: Synthesis of a 43-nucleotide sequence similar to the 3'-half molecule of an Escherichia coli formylmethionine tRNA1"<br>* Pages 7846-7847 *<br>--- | 1-8,13, 15 | |
| Y | TETRAHEDRON LETTERS, vol. 28, no. 41, 1987, pages 4897-4900, Pergamon Journals Ltd, Oxford, GB; T. SUDHAKAR RAO et al.: "Solid phase synthesis of the 3'-terminal nonadecaribonucleoside octadecaphosphate sequence of yeast alanine transfer ribonucleic acid"<br>* En entier *<br>--- | 1-8,13, 15 | **DOMAINES TECHNIQUES RECHERCHES (Int. Cl.5)**<br>C 07 H 21/00<br>C 07 H 19/00 |
| Y | WO-A-8 705 906 (KING'S COLLEGE LONDON)<br>* En entier *<br>----- | 1,2 | |

Le présent rapport a été établi pour toutes les revendications

| Lieu de la recherche | Date d'achèvement de la recherche | Examinateur |
|---|---|---|
| LA HAYE | 15-09-1989 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)